# EUROPEAN PATENT APPLICATION

(11) **EP 4 830 762 A1**
(43) Date of publication of application: **28.10.2026**
(21) Application number: 25382405.6
(22) Date of filing: 22.04.2025
(51) Int. Cl.: A61N 1/04, A61B 5/296, A61B 5/389, A61N 1/36

(54) **SYSTEM FOR SYNCHRONIZED RESPIRATORY MUSCULAR ELECTRICAL STIMULATION OF A PATIENT**

(71) Applicant: Tesai Care Sociedad Limitada, 08003 Barcelona (ES); Fundació Hospital Universitari Vall d'Hebron - Institut de Recerca, 08035 Barcelona (ES); Fundació Institut d'Investigació i Innovació Parc Taulí (I3PT), 08208 Sabadell (Barcelona) (ES)
(72) Inventor: FERREIRA QUARTINO, Sofía, 08003 Barcelona (ES); LIZAMA VELÁZQUEZ, Laura, 08003 Barcelona (ES); ZARRANZ BOZAL, Nora, 08003 Barcelona (ES); ROCA GAS, Oriol, 08003 Barcelona (ES); SÁNCHEZ GALARRAGA, Jaime, 08003 Barcelona (ES); GARCÍA GARCÍA DE ACILU, Marina, 08208 Sabadell (ES); RUIZ ÁVILA, Luis Antonio, 08003 Barcelona (ES)
(74) Representative: Hoffmann Eitle

(57) **Abstract**

The invention relates to a system for synchronized respiratory muscular electrical stimulation of a patient. The system comprises: a transcutaneous electrostimulation of the respiratory muscles (TERM) system comprising one or more skin electrodes configured to be placed over the respiratory muscles of the patient; at least one sensor for sensing the breathing state of the patient; and a processor configured to receive the breathing state of the patient from the at least one sensor and actuate the TERM system when the breathing state is in the inspiration phase. Each of the one or more skin electrodes are multifield electrodes comprising an anode and a cathode; the processor is further configured to actuate the one or more multifield skin electrodes at a current intensity of at least 20 mA The invention also relates to a method and computer program product thereof.

## Description

### Technical field of the invention

The present invention belongs to the field of muscular therapy more particularly to electrical respiratory muscular therapy. Particularly, the present invention relates to a system for synchronized respiratory muscular electrical stimulation of a patient, to a method thereof and to a computer program product thereof.

### Background of the invention

Intensive Care Unit (ICU) patients require constant supervision from medical staff to evaluate their vital signs. In a significant number of cases, the patients require mechanical ventilation (MV) assistance during long periods of time in order to achieve appropriate oxygen saturation levels.

Invasive MV is a common procedure in ICUs, applied to approximately 33% of critically ill patients to support respiration while avoiding excessive respiratory muscle work. Weaning from mechanical ventilation usually presents a problem due to the muscles weakness, which represents a reduction in the level of force generation at a given point in time [2].

Diaphragm weakness is a leading cause of difficult weaning from mechanical ventilation. A wide variety of factors can give rise to acute or chronic diaphragm weakness in critically ill patients, including pre-admission injury, sepsis, medications, and multiorgan dysfunction syndrome. Also, there are other potentially contributing factors such as sedatives or analgesics. However, mechanical ventilation per se can cause acute diaphragm injury since it may further increase muscle weakness developing a loss of the capability to generate skeletal muscle force and/or velocity that is accompanied by recovery during rest, also called muscle fatigue. Consequently, the progressive development of diaphragm atrophy during the early course of mechanical ventilation predicts prolonged ventilation and an increased risk of complications of acute respiratory failure.

Several systems and methods have been designed in order to improve the diaphragm condition during the mechanical ventilation period by electrically or mechanically stimulating the muscle. These usually imply the use of invasive electrodes inserted onto the respiratory muscles in order to achieve proper nerve stimulation. However, many ICU patients due to their medical condition are not eligible for invasive stimulation. Non-invasive solution require the use of higher intensities to reach the desired stimulation but this involves inevitable electrical noise (as shown in Example 1) that affects and distorts the measurements of biometric signals essential for ICU monitoring of the patient.

Therefore, all these solutions, involve a complex system that interfere physically and electrically with the day-to-day work in the ICU. For example, needing to constantly monitor the breathing sensor to ensure it works appropriately, involves further work for the medical staff, increasing the chances of a mistake, while it further uses space around the patient, which, depending on their state can be already saturated. Also, the electrostimulation provided by the already disclosed systems, due to their power and location (around the chest of the patient), electrically interfere with the reading from other devices such as electrocardiograms (ECG). The current provided by present solutions are reflected in these monitoring signals as distortions, that either interfere with the ECG signals, making therefore electrostimulation incompatible with these type of monitorization, or require manual supervision of the monitorization, as automatic systems are confused by these distortions, interpreting them as hazards for the patient. Thus, current solutions ether increase the resources needed for each patient or are incompatible with ICU monitoring systems.

There is therefore a need for a system for a non-invasive synchronized respiratory muscular electrical stimulation of patients which can be effectively applied within the ICU complex requirements in terms of space, time, staff and automatic monitorization of the patients. Preferably, there is a need for a calibratable system for a non-invasive synchronized respiratory muscular electrical stimulation of patients which can be effectively applied within the ICU complex requirements in terms of space, time, staff and automatic monitorization of the patients.

### Summary of the invention

A first aspect of the invention relates to a system for synchronized respiratory muscular electrical stimulation of a patient. The system comprises: a transcutaneous electrostimulation of the respiratory muscles (TERM) system comprising one or more skin electrodes configured to be placed over the respiratory muscles of the patient; at least one sensor for sensing the breathing state of the patient; and a processor configured to receive the breathing state of the patient from the at least one sensor, and actuate the TERM system when the breathing state is in the inspiration phase; wherein each of the one or more skin electrodes are multifield electrodes comprising an anode and a cathode, and wherein the processor is further configured to actuate the one or more multifield skin electrodes at a current intensity of at least 20 mA..

In a preferred embodiment of the first aspect of the invention, the processor is further configured to actuate the one or more multifield skin electrodes at a current intensity comprised between 20 mA and 100 mA.

In a more preferred embodiment, when the processor is configured to actuate the TERM system when the breathing state is in the inspiration phase the processor is further configured to actuate the one or more multifield skin electrodes at a current intensity comprised between 20 mA and 60 mA.

In another preferred embodiment, the processor is further configured to actuate the TERM system (10) when the breathing state is in the expiration phase.

In a more preferred embodiment, when the processor is configured to actuate the TERM system when the breathing state is in the expiration phase the processor is further configured to actuate the one or more multifield skin electrodes at a current intensity comprised between 40 mA and 100 mA.

In another preferred embodiment of the first aspect of the invention, the system further comprises at least one electromyography (EMG) sensor, the at least one EMG sensor configured to measure the activity of one or more of the respiratory muscles of the patient, preferably wherein the at least one EMG sensor is a superficial EMG sensor. The processor is further configured to actuate the TERM system considering the activity of one or more of the respiratory muscles of the patient as measured by the EMG sensor.

In a more preferred embodiment, the processor is further configured to determine the fatigue of the respiratory muscles of the patient from the activity registered by the at least one EMG sensor; and the processor is further configured to actuate the TERM system considering the fatigue status of the respiratory muscles of the patient.

In another preferred embodiment of the first aspect of the invention, the TERM system is configured to apply at least two electrical stimuli with an intensity that distorts the one or more biometric measurements performed by one or more monitoring devices and with a period between consecutive electrical stimuli of at least 200 milliseconds.

In another preferred embodiment of the first aspect of the invention, the one or more biometric measurements require a minimum measurement period to obtain a parameter of interest and the period between consecutive electrical stimuli is extended by an additional period corresponding to the measurement period the one or more biometric measurements require.

In another preferred embodiment of the first aspect of the invention, the intensity that distorts the one or more biometric measurements performed by one or more monitoring devices is at least of 20mA.

In another preferred embodiment of the first aspect of the invention, the biometric measurement is an electrocardiogram and the monitoring device an electrocardiogram monitoring device.

In another preferred embodiment of the first aspect of the invention, the at least two electrical stimuli is further adapted based on mechanical ventilated-related variables, preferably wherein the mechanical ventilated-related variables comprise any of: airway pressure (Paw), flow, and volume pressure.

In another more preferred embodiment of the first aspect of the invention, the TERM system is configured to actuate the one or more multifield skin electrodes using an anode field and cathode field with an area between 10 and 50 cm², preferably 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 cm2.

A second aspect of the invention relates to a computer implemented method for synchronized electrical stimulation with a system for synchronized respiratory muscular electrical stimulation of a patient according to any one of the embodiments of the first aspect of the invention. The method comprises:
a) receiving information regarding the breathing state of the patient from a sensor connected to a mechanical ventilator,
b) determining from the information if the breathing state corresponds to a selected breathing phase of the patient wherein the selected breathing phase is selected from any of: inspiration phase and/or expiration phase;
c) when the breathing state corresponds to the selected breathing phase of the patient, indicating to the processor that the breathing state corresponds to the selected breathing phase of the patient.

A fourth aspect of the invention relates to a computer program product comprising instructions configured to perform the method according to any of the second and/or the third aspect of the invention.

### Brief description of the drawings

To enable a better understanding of the present disclosure, and to show how the present disclosure may be carried out, reference will now be made, by way of example only, to the accompanying schematic drawings, wherein:
Fig. 1 shows a diagram of a system for synchronized respiratory muscular electrical stimulation of a patient according to one or more embodiments of the invention.
Fig. 2 shows the location over the patient's body of (a) the stimulating electrodes and (b) the EMG registering electrodes according to one or more embodiments of the invention.
Fig. 3 shows a diagram of a method for synchronized diaphragmatic electrical stimulation according to one or more embodiments of the invention.
Fig. 4 (a-d) shows EMG recording samples of 4 different registers being distorted and then recovered according to one or more embodiments of the invention.
Fig. 5 shows a table for the transdiaphragmatic pressure delta between recordings with and without TERM with an observational analysis (a) and with a window analysis (b) when the TERM is applied according to one or more embodiments of the invention.
Fig. 6 shows a multifield skin electrode for (A) inspiration TERM stimulation and (B) expiration TERM stimulation according to one or more embodiments of the invention.
Fig. 7 shows another diagram of a method for synchronized diaphragmatic electrical stimulation according to one or more embodiments of the invention.
Fig. 8 shows another diagram of a method for synchronized diaphragmatic electrical stimulation according to one or more embodiments of the invention.
Fig. 9 shows a table for the field and current range that generate a detectable respiratory muscle contraction without distortion or with the least distortion of one or more biometric measurements performed on 13 subjects by one or more monitoring devices according to one or more embodiments of the invention.
Fig. 10 shows a table for the minimum and maximum contraction current and the relationship between them according to one or more embodiments of the invention.
Fig. 11 shows a graph for the relationship factor between the threshold intensity and maximum contraction intensities of all patients for each body mass index according to an implementation of one or more embodiments of the invention.
Fig. 12 shows images of (a) electrode positions to stimulate the diaphragm and intercostal muscles, (b) first electrode positions to stimulate the back portion of the diaphragm and (c) second electrode positions to stimulate the back portion of the diaphragm muscles, according to one or more embodiments of the invention.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "electrode" refers to a conductor through which electricity enters or leaves an object, substance or region. In the context of the present invention, the term "electrode" preferably refers to a conductor through which electricity enters or leaves a patient. In some embodiments, the term electrode may refer to any of a working, counter, or reference electrode, indistinctively.

The term "respiratory muscle" refers to a muscle involved, either directly or indirectly, in the breathing process. This comprises both inspiration and expiration muscles.

The term "breathing state" refers to a state of the breathing process of mammals, including inspiration and expiration. In the present disclosure, the term "inspiration" may be referred to as "breathing in" or "inhalation"; and "expiration" may be referred to "breathing out", or "exhalation", indistinctively.

The term "biometric measurement" refers to any measurement of a parameter or signal produced by a living body. This comprises also elicited signals such as evoked potentials. In the context of the present invention, it preferably refers to measurements associated to clinically relevant parameters, such as electroencephalography (EEG), electrocardiography (ECG) or electromyography (EMG).

The term "anthropometric parameters" refers to parameters associated to the physical proportions and measurements of a human. In the context of the present invention, it preferably refers to the physical proportions and measurements of a patient, including Body Mass Index (BMI), skin folds, abdominal circumferences, skin temperature, blood flow, bioimpedance measurements, rheobase and chronaxie.

The term "demographic parameters" refers to parameters associated with the population-related data of a human. In the context of the present invention, it preferably refers to the parameters including age or sex.

The term "rheobase" refers to the minimum current required to depolarize a nerve given an infinite duration of stimulation. In the context of the present invention, it preferably refers to the minimum current required to depolarize a nerve associated to a particular muscle of a patient, given an infinite duration of stimulation.

The term "chronaxie" refers to the duration of current required to depolarize a nerve to threshold when the current is two times the rheobase. In the context of the present invention, it preferably refers to the duration of current required to depolarize a nerve to threshold associated to a particular muscle of a patient when the current is two times the rheobase.

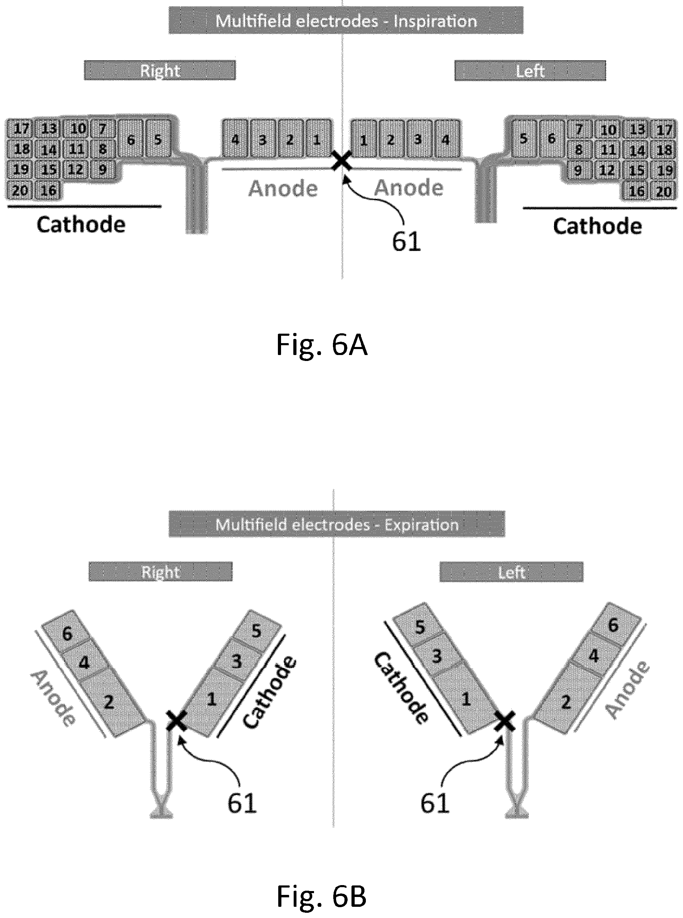

The term "multifield electrodes", refers to electrodes that are divided into a certain number of fields or pads that can be activated independently and with different current amplitudes. They may be also referred to as "array electrodes" .It is noted that although the term "array" is used, the multifield electrode may have a matrix-like configuration as shown for example in Fig. 6A and 6B.

The term "field" in the context of the present invention when referred to an electrode, such as an anode or cathode, within a multifield electrodes unit, refers to the particular section or pad of the multifield electrode associated to said electrode.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

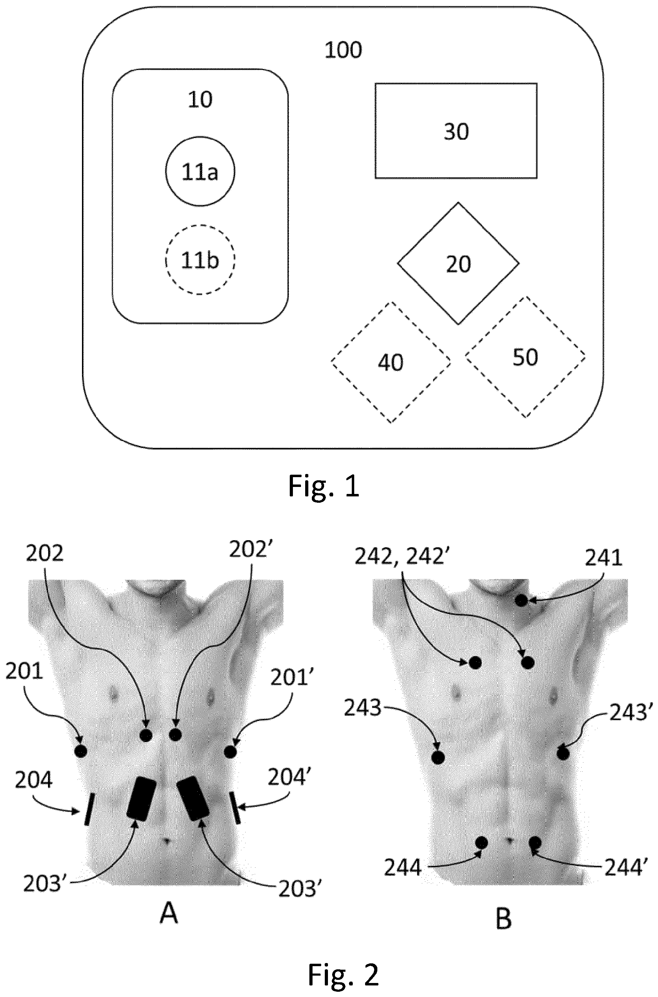

A first aspect of the invention relates to a system 100 or synchronized respiratory muscular electrical stimulation of a patient. As shown with respect to Fig. 1, the system comprises a transcutaneous electrostimulation of the respiratory muscles (TERM)system 10, at least one sensor 20 for sensing the breathing state of the patient and a processor 30.

The TERM system 10 comprises one or more skin electrodes 11 configured to be placed over the respiratory muscles of the patient. The TERM system 10 may be any type of system configured to provide respiratory muscle electrical stimulation as know by the skilled person. Thus, the TERM system 10 may be configured to apply different types of stimulations, through one or more electrodes, and may be configured to be adapted to also work with other different types of electrodes, including non-invasive electrodes 11, preferably superficial skin electrodes 11. While the TERM system 10 may be mainly designed for respiratory muscle electrostimulation, in the context of the present invention, the TERM system may be also configured to be able to further stimulate other type of muscles different from respiratory muscles. In some embodiments, the TERM system 10 may comprise many other element such as a processor, signal processing units, oscillators, a power unit, communication modules and any other elements that may further contribute to generation and/or provision of the muscle electrical stimulation.

The one or more skin electrodes 11, such as 11a or 11b, may comprise different types of skin electrodes. Thus, the one or more electrodes 11 may be dry or wet electrodes, such as gel electrodes. The one or more electrodes 11 may also be reusable or disposable and may comprise different materials such as Ag, Ag-AgCl, Pt, Au, among others known by the skilled person. The one or more electrodes 11, may also take different shapes and sizes, including shapes and sizes designed for particular locations of the skin or muscles to be stimulated. When the TERM system 10 comprises more than one skin electrodes 11, the skin electrodes 11 may be different types of electrodes, and each electrode may comprise different materials, shapes and sizes.

It is noted that the one or more skin electrodes 11 are configured to be placed over the respiratory muscles of the patient in terms of types, material, shape and size. Thus, a particular skin electrode may be of a particular type, comprise a particular material, and have a particular shape and size according to the properties of the muscle the skin electrode 11 is configured to stimulate. For such electrode parameters the muscle size, fibre types, depth form the skin and location may be considered.

The at least one sensor 20 for sensing the breathing state of the patient may be one of many different types of sensors. Thus, the breathing sensor 20 may be a flow sensor or a switch associated to the mechanical ventilation system output, or may be a chest diameter sensor configured to detect inspirations and expirations by the patient, among others. Thus, in some embodiments and as will be explained further below, the at least one sensor 20 for sensing the breathing state of the patient may be comprised within the mechanical ventilation system. It is also noted that the system may comprise more than one sensor 20 that may work as a security layer through redundancy or may be configured to detect different breathing states.

It is noted that the processor 30 may comprise one or more processing units, such as a microprocessor, GPU, CPU, multi-core processor or the like. In some embodiments, it may be associated to a memory, wherein the memory may comprise one or more volatile or nonvolatile memory devices, such as DRAM, SRAM, flash memory, read-only memory, ferroelectric RAM, hard disk drives, floppy disks, magnetic tapes, optical disks or the like.

The processor 30 is configured to receive the breathing state of the patient from the at least one sensor 20. This may be done through a wired connection, such as a data communication cable, or through a wireless connection, such as Bluetooth, Wi-Fi, NFC, ZigBee. The skilled person may envisage many other alternative ways, in which the processor 30 is configured to receive the breathing state of the patient from the at least one sensor 20, including different protocols and ad-hoc communication implementations.

The processor 30 is also configured to actuate the TERM system 10 when the breathing state is in the inspiration phase and/or the expiration phase. As defined above, from the breathing state the processor 30 can determine if the patient is in the inspiration phase or the expiration phase. Therefore, the processor 30 can be configured to actuate the TERM system 10 when the breathing state is in either one of the phases, or in both phases. This may be selected by a user according to the patient needs. The actuation of the TERM system 10 when the breathing state is in the inspiration phase will stimulate the inspiration muscles while the actuation of the TERM system 10 when the breathing state is in the expiration phase will actuate the expiration muscles. Therefore, preferably the one or more skin electrodes 11 are preferably configured to be placed over the inspiratory and/or expiratory respiratory muscles of the patient, respectively. In some embodiments, the processor 30 may be the processor of the TERM system 10.

In a preferred embodiment of the first aspect of the invention, the TERM system 10 is configured to apply at least two electrical stimuli with an intensity that distorts the one or more biometric measurements performed by one or more monitoring devices and with a period between consecutive electrical stimuli of at least 200 milliseconds.

The minimum intensity or amplitude of the at least two electrical stimuli that distorts the one or more biometric measurements performed by one or more monitoring devices, depends on the patient's characteristics interfering with the stimuli propagation and on the monitoring device point of registration and the one or more biometric measurement monitored.

When an electrical stimulus is applied subsequently to the previous one, the distortion of the electrical stimulus can be continuous to the distortion produced by the previous electrical stimulus. Thus, it has been shown in Example 2 that a period of at least 200 milliseconds is sufficient for the signal of the one or more biometric measurement being monitored to recover from the distortion of the previous electrical stimulus. Thus, even in the at least two electrical stimuli are applied with an intensity that distorts the one or more biometric measurements, due to the minimum period of at least 100ms between the at least two electrical stimuli, the signal of the one or more biometric measurement monitored is recovered between the stimuli.

Advantageously, this means that the continuous provision of electrical stimuli by the TERM system 10 does not interfere with the monitoring of the one or more biometric measurements performed by one or more monitoring devices. In turn, this means that an effective synchronized respiratory muscular electrical stimulation can be applied to a patient without interfering with the routine of ICU, thus being a seamless system for the staff. It is noted that although the expression "biometric measurements" is plural, as the one or more monitoring devices usually monitor more than one measurement, the present invention can be applied mutatis mutandis when there is only one biometric measurement being monitored.

As shown in Example 2, a period of at least 200 milliseconds between the at least two electrical stimuli is sufficient to allows the signal to recover appropriately such that the distortion produced by the stimuli does not interfere with the one or more biometric measurements.

It is noted that the period refers to the time lapse between two consecutive electrical stimuli. However, when the TERM system 10 is configured to provide more than two electrical stimuli, the period refers to the time lapse between each of any two consecutive electrical stimuli. It is also noted that when the TERM system 10 is configured to provide more than two electrical stimuli, the period between each two consecutive electrical stimuli may be different, as long as it is of at least 200 milliseconds.

It is noted that Fig. 1 comprises many other optional elements that are not comprised within all the embodiments of the first aspect of the invention, marked with dashed borders. For example, Fig. 1 shows a system 100 with a second skin electrode 11b. However, it is noted that according to the first aspect of the invention the system may comprise only one skin electrode 11a. Fig. 1 also shows a system 100 with other sensors 40, 50, different to the at least one sensor 20 for sensing the breathing state of the patient, as will be described further below in some embodiments.

According to another preferred embodiment of the first aspect of the invention, the one or more biometric measurements require a minimum measurement period to obtain a parameter of interest, and the period between consecutive electrical stimuli is extended by an additional period corresponding to the measurement period the one or more biometric measurements require. Advantageously, this allows for the one or more biometric measurements to be effectively used to obtain the parameter of interest while the synchronized respiratory muscular electrical stimulation is provided.

The parameter of interest may be any parameter of clinical relevance obtained through the one or more biometric measurement. For example, the heart rate (HR) is a parameter of interest obtained through the electrocardiogram (ECG). The minimum measurement period to obtain the HR through an ECG is 1/HR, although 2/HR is usually desirable to contrast inter-beats parameters. Likewise, the Root Mean Square (RMS) or the Mean Frequency (MF) are parameters obtained through the electromyogram (EMG) which give information about muscle fatigue. The minimum measurement period to obtain those values through an EMG is the duration of 1 contraction, although 2 contractions is useful to observe the evolution. The duration of the contraction may be stablished in a dynamic way, for example through an EMG intensity threshold able to determine the number of contractions performed since a particular point in time.

It is however noted that not all biometric measurements require a minimum measurement period to obtain a parameter of interest. For instance, the EEG is a continuous monitored biometric measurement that usually does not have any parameter of interest to monitor apart from the signal itself. It is therefore noted that in some other embodiments of the first aspect of the invention, the period between consecutive electrical stimuli may not be extended by an additional period whatsoever. Thus, this extension of the period between consecutive electrical stimuli is optional for one or more embodiments of the first aspect of the invention and is not essential for the invention herein described.

According to another preferred embodiment of the first aspect of the invention, the intensity that distorts the one or more biometric measurements performed by one or more monitoring devices is at least of 20mA.

Advantageously, and as shown with respect to Example 3, it has been found that this intensity is enough to provide an effective stimulation of the respiratory muscles. When at least two electrical stimuli with an intensity of at least 20mA are provided with a period of at least 100ms, the distortion of each electrical stimulus does not interfere with the appropriate monitorization of the one or more biometric measurements signals. Thus, the muscular dystrophia is effectively prevented, while the one or more biometric measurements can be carried out as usual in the UCI environment.

According to a more preferred embodiment of the first aspect of the invention, the intensity that distorts the one or more biometric measurements performed by one or more monitoring devices is at least of 60mA.

Advantageously, and as shown Example 2, it has been found that this intensity provides a further effective stimulation of the respiratory muscles. When at least two electrical stimuli with an intensity of at least 60mA are provided with a period of at least 100ms, the distortion of each electrical stimulus does not interfere with the appropriate monitorization of the one or more biometric measurements signals. Thus, the respiratory muscles are more effectively stimulated, while the one or more biometric measurements can be carried out as usual in the UCI environment.

In another preferred embodiment according to any one of the embodiments of the first aspect of the invention, the period is of at least 225, more preferably 250, even more preferably 300 milliseconds.

In a more preferred embodiment of the first aspect of the invention, the TERM system (10) is configured to apply at least two electrical stimuli with an intensity that distorts the one or more biometric measurements performed by one or more monitoring devices and with a period between consecutive electrical stimuli of at least 325 milliseconds.

Advantageously, and as shown in Example 2, this further helps providing enough time for the signal of the one or more biometric measurements being monitored to recover from the distortion of the previous electrical stimulus. This further prevents the electrical stimulation of the respiratory muscles from interfering with the required monitoring of the one or more biometric measurements within the ICU environment.

According to a more preferred embodiment of the first aspect of the invention, the period is of at least 1 second.

Advantageously, and as shown in Example 2, this further ensures that the signal of the one or more biometric measurements being monitored to recover from the distortion of the previous electrical stimulus. This further prevents the electrical stimulation of the respiratory muscles from interfering with the required monitoring of the one or more biometric measurements within the ICU environment. In another preferred embodiment according to any one of the embodiments of the first aspect of the invention, when the processor configured to actuate the TERM system 10, when the breathing state is in the inspiration phase the one or more electrodes 11 are configured to be placed over the front, lateral and/or back region of the torso of the patient.

It is noted that the inspiration phase of breathing involves many muscles that can be stimulated from many different parts of the body, including the front , lateral and/or back region of the torso of the patient. Thus, by configuring the TERM system 10to comprise one or more electrodes 11 configured to be placed over the front , lateral and/or back region of the torso of the patient, the system is further configured to non-invasively stimulate the inspiratory respiratory muscles.

More preferably, the one or more electrodes 11 are configured to be placed over the skin on the parts of the torso skin closest to the inspiratory respiratory muscles, as will be described now.

According to a more preferred embodiment of the first aspect of the invention, and as shown with respect to Fig. 2A, the one or more electrodes 11 are configured to be placed over the intercostal muscles, more preferably over the external intercostal muscles 201, 201' of the patient. For example, and as shown in Fig. 2A, the one or more electrodes 11 may be located in the 7^{th} intercostal space in the mid-axillary line.

Advantageously, the TERM system 10 comprises one or more electrodes 11 further configured to be placed non-invasively over a set of muscles which have a relevant role in the inspiratory phase of breathing.

It is noted that according to this more preferred embodiment, the one or more electrodes 11 may be configured to be placed over different regions of the torso of the patient as long as they are configured to be placed over any portion of the external intercostal muscles.

It is noted that Fig. 2A shows many other electrode positions over the torso of the patient for which the one or more electrodes 11 may not be configured to be placed over, according to one or more embodiments of this preferred embodiment. Thus, many electrode positions may be alternative and/or may be used for other breathing states. It is also noted that that according to Fig. 2A, there are two electrode positions for each type of electrode position, however, in some embodiments according to this preferred embodiment, the TERM system 10 may comprise only one electrode 11 or more than two electrodes 11 configured to be placed over such region. Finally, it is noted that most regions 201-205 are indicated as a circle while some are more elongated. However, the embodiments according to this preferred embodiment are not limited with respect to the particular shape of each region 201-205 and may be different from those shown in Fig. 2A.

In another more preferred embodiment according to any one of the embodiments of the first aspect of the invention, and as shown with respect to Fig. 2A , the one or more electrodes 11 are configured to be placed over the diaphragm muscle 202, 202' of the patient.

Advantageously, the TERM system 10 comprises one or more electrodes 11 further configured to be placed non-invasively over a set of muscles which have a relevant role in the inspiratory phase of breathing. It is noted that according to this more preferred embodiment, the one or more electrodes 11 may be configured to be placed over different regions of the torso of the patient, as long as they are configured to be placed over any portion of the diaphragm muscle.

In another preferred embodiment according to any one of the embodiments of the first aspect of the invention, when the processor configured to actuate the TERM system 10, when the breathing state is in the expiration phase the one or more electrodes 11 are configured to be placed over the front, lateral and/or back region of the torso of the patient.

It is noted that the expiration phase of breathing involves many muscles that can be stimulated from many different parts of the body, including the front , lateral and/or back region of the torso of the patient. Thus, by configuring the TERM system 10to comprise one or more electrodes 11 configured to be placed over the front , lateral and/or back region of the torso of the patient, the system is further configured to non-invasively stimulate the expiratory respiratory muscles.

More preferably, the one or more electrodes 11 are configured to be placed over the skin on the parts of the torso skin closest to the expiratory respiratory muscles, as will be described now.

According to a more preferred embodiment of the first aspect of the invention, and as shown with respect to Fig. 2A, the one or more electrodes 11 are configured to be placed over the abdominal 203, 203', oblique, internal oblique and/or transversus muscles 204, 204' of the patient.

Advantageously, the TERM system 10 comprises one or more electrodes 11 further configured to be placed non-invasively over a set of muscles which have a relevant role in the expiratory phase of breathing.

It is noted that according to this more preferred embodiment, the one or more electrodes 11 may be configured to be placed over different regions of the torso of the patient, such as in the iliac spine anterosuperior and inclined obliquely along the costal margin and towards the xiphoid process 203, 203' or at the intersection between the posterior middle axillary line and the iliac crest and at an angle to the middle axillary line 204, 204', as long as they are configured to be placed over any portion of the abdominal, oblique, Internal oblique and/or transversus muscles.

In another preferred embodiment according to any one of the embodiments of the first aspect of the invention, and as shown with respect to Fig. 1, the system 100 further comprises at least one electromyography (EMG) sensor 40, the at least one EMG sensor 40 configured to measure the activity of one or more of the respiratory muscles of the patient.

An EMG sensor is a sensor configured to register the electrical activity of the muscles of the patient. It is noted that the EMG sensor 40 may be of different types including bipolar electrodes. Advantageously, a system 100 comprising at least one EMG sensor can further use the activity of one or more of the respiratory muscles of the patient measured by the EMG sensor to modulate the actuation of the TERM system, such as the at least two electrical stimuli provided by the TERM system 10. This may be performed for example through one or more multifield skin electrodes (11a, 11b). Hence, the processor (30) is further configured to actuate the TERM system (10) considering the activity of one or more of the respiratory muscles of the patient as measured by the EMG sensor, This in turn can be used to infer different properties of the patient, such as the muscle fatigue, respiratory cycles, maximum contraction of the muscle, and/or the contraction speed of muscle fibres which can be used to adapt the stimuli properties such as amplitude and/or periods. For example, if muscle fatigue is detected, it can be stablished that a resting period of a certain amount of time, such as a breathing cycle shall be stablished until the fatigue is diminished or until a predetermined time threshold is reached. Alternatively, or additionally, the amplitude of the stimulation can be diminished to reduce the fatigue produced by new stimulations.

It is noted that from the above disclosure, the skilled person may envisage many other different strategies the TERM system 10 may be configured to ensure muscle fatigue is under control, such as alternating different series of stimuli with series of resting. Likewise, series of high intensity electrical stimuli may be alternated with series of low intensity electrical stimuli. These may be even combined. All of these different possible combinations of period and intensity variations that may be envisaged from the above are also comprised within the first aspect of the invention.

This further helps to provide an appropriate stimulation in complex environments and with complex patients, such as ICU patient, which in most cases are not autonomous and/or unconscious and cannot provide feedback on the fatigue. If the respiratory muscles being stimulated are fatigued, the recovery and/or tonifying of the muscle can be compromised. It is therefore a very preferred option to further include means to monitor the muscle fatigue while the electrical stimulation of the respiratory muscles is being applied.

It is noted that in Fig. 1 there is only one EMG sensor 40 depicted, however, in other embodiments of this preferred embodiment of the first aspect of the invention, the system 100 may comprise more than one EMG sensor. It is also noted, that when the system 100 comprises more than one EMG sensor, the EMG sensors may be different between them. The system 100 may comprise more than one EMG sensor to measure the activity different respiratory muscles and/or for redundancy purposes.

According to a more preferred embodiment of the first aspect of the invention, the at least one EMG sensor 40 is a superficial EMG (sEMG) sensor. Advantageously, this further ensures the system 100 to be non-invasive when the EMG sensor is in use, which in turn further simplifies the use in complex environments and critical patients, such as those in the ICU.

In a furthermore preferred embodiment of the first aspect of the invention, and as shown with respect to Fig. 2B, the at least one EMG sensor 40 is configured to be located over the sternocleidomastoid 241, intercostal muscles 242, diaphragm 243, and/or rectus anterior muscles 244. Advantageously, these muscles are easily accessible in patients located in ICU environments.

For example, and as shown in Fig. 2B, the at least one EMG sensor 40 may be configured to be located over the sternocleidomastoid 241 at 20% of the distance between the mastoid apophysis and the sternal notch and over the line that connects them. In another example, the at least one EMG sensor 40 may be configured to be located over the intercostal muscles 242 at the 2^{nd} intercostal space, bilaterally on the parasternal line. In yet another example the at least one EMG sensor 40 may be configured to be located over the diaphragm 243 at the inferior costal margin, bilaterally in the midclavicular line. In a further example, the at least one EMG sensor 40 may be configured to be located over the rectus anterior muscles 244 at the midclavicular line, at the level of the umbilicus.

It is noted that Fig. 2B shows many electrode positions over the torso of the patient for which sEMG sensor 40 may not be configured to be placed over, according to one or more embodiments of this preferred embodiment. Thus, many electrode positions may be alternative and/or may be used for other muscles or locations. It is also noted that that according to Fig. 2B, there are one or two sEMG sensor 40 positions for each type of electrode position, however, in some embodiments according to this preferred embodiment, the TERM system 10 may comprise only one sEMG sensor 40 or more than two sEMG sensors 40 configured to be placed over such region. Finally, it is noted that the regions 241-244 are indicated as a circle. However, the embodiments according to this preferred embodiment are not limited with respect to the particular shape of each region 241-244 and may be different from those shown in Fig. 2B. Also, when the sEMG sensor is a bipolar sensor, each of the regions shown in Fig. 2B may represent the location of two electrodes.

In another furthermore preferred embodiment of the first aspect of the invention the EMG sensor comprises a reference electrode. Preferably over the breastbone, on the ankle or the wrist of the patient. Advantageously, these locations provide good reference elements as they have easy access to bones and are far away from the sEMG electrodes.

Different patients have different physiological characteristics which implies that they require the provision of different electrical stimuli in order to effectively stimulate the respiratory muscles and prevent the muscular dystrophia. In another preferred embodiment according to any one of the embodiments of the first aspect of the invention, the TERM system 11 is further configured to be actuated, such as to apply the at least two electrical stimuli, adapted to the patient based on patient's physiology, anthropometric and/or demographic parameters of the patient. The at least two electrical stimuli are adapted to the patient by adapting the amplitude, frequency, shape, and/or duration of the at least two stimuli. This may be performed for example through one or more multifield skin electrodes (11a, 11b).

Advantageously, this further configures the system 100 to be able to provide effective stimulation adapted for the patient. Therefore, given the patient's physiology, anthropometric and/or demographic parameters the appropriate amplitude frequency, shape, and/or duration of the two or more stimuli required for the patient can be effectively provided.

According to a more preferred embodiment of the first aspect of the invention, the anthropometric parameters comprise one or more parameters selected from: Body Mass Index (BMI), skin folds, abdominal circumferences, skin temperature, blood flow, bioimpedance measurements, rheobase and chronaxie.

The BMI, the skin folds and the abdominal circumferences are correlated with the amount of body fat and can be therefore used to estimate the body fat of the user. As the fat is stored between the skin and the muscles, the body fat interferes with the provision of the at least two stimuli to the respiratory muscles. Thus, knowing the BMI, the skin folds and/or the abdominal circumferences of the patient can be used to actuate the TERM system, such as to apply the at least two electrical stimuli, adapted to the patient. This may be performed for example through one or more multifield skin electrodes (11a, 11b).

The skin temperature is correlated with the skin blood flow, which in turn is correlated with the electrical resistance of the skin. Thus, knowing the skin temperature and/or the skin blood flow of the patient can be used to apply the actuate the TERM system, such as to at least two electrical stimuli adapted to the patient.

The bioimpedance is indicative of the resistance of the body to electricity. Thus, knowing the bioimpedance of the patient can be used to actuate the TERM system, such as to apply the at least two electrical stimuli adapted to the patient.

The rheobase is the smallest current amplitude that when applied for an infinite amount of time would result in muscle fibre activation and the chronaxie is the time duration of the current necessary for fibre activation with a stimulus twice that of the rheobase. These variables provide information about the response of the muscle fibres under different stimulation times and current amplitudes. Thus, knowing the rheobase and/or the chronaxie of the patient can be used to apply the actuate the TERM system, such as to at least two electrical stimuli adapted to the patient.

It is noted that according to this more preferred embodiment of the first aspect of the invention any combination of the BMI, skin folds, abdominal circumferences, skin temperature, blood flow, bioimpedance measurements, rheobase and/or chronaxie may be used in order to actuate the TERM system, such as to apply the at least two electrical stimuli adapted to the patient. It is also noted that any of the above-mentioned parameters may be used in different combinations to adapt the least two electrical stimuli to different respiratory muscles.

According to another more preferred embodiment of the first aspect of the invention, the system 100 further comprises means 50 for determining one or more anthropometric parameters selected from: skin temperature, blood flow, bioimpedance measurements, rheobase and chronaxie.

Advantageously, a system 100 comprising means 50 for determining one or more anthropometric parameters selected from: skin temperature, blood flow, bioimpedance measurements, rheobase and chronaxie can further determine these anthropometric parameters without relying on external systems, which further simplifies the system 100 and makes it simpler, easier to control and less intrusive for the medical staff. For example, the means 50 for determining the one or more anthropometric parameters can be autonomously connected once turned on, which makes the use of the devices seamless to the medical staff, further facilitating the use of the system 100 for synchronized respiratory muscular electrical stimulation in the ICU conditions.

In another preferred embodiment according to any one of the embodiments of the first aspect of the invention, the at least one sensor 20 for sensing the breathing state of the patient is comprised within a mechanical ventilation system. The mechanical ventilation system may comprise a mechanical ventilator and its accessories that may comprise within a typical setup, including the nozzles and air intakes. The skilled person may note the different accessories a mechanical ventilator may be attached to in an ICU setup, all of which are regarded to be comprised within a mechanical ventilation system in the present disclosure.

Advantageously, when the at least one sensor 20 for sensing the breathing state of the patient is comprised within the mechanical ventilation system attached to the patient, there is no need for an additional sensor in order to determine the berthing state of the patient. This reduces costs, complexity of the system as the sensor of the mechanical ventilation system is reused and the resources requires to the staff, including the supervision of an additional sensor. The at least one sensor 20 may be comprised within the mechanical ventilation system which is attached to the patient in different ways. For example, the status of the mechanical ventilation system, or the flow meter of the mechanical ventilation system, or any other parameters or variable of the mechanical ventilation system may be used as the sensor 20. From this disclosure, the skilled person may envisage many other different ways in which the at least one sensor 20 for sensing the breathing state of the patient may comprised within the mechanical ventilation system attached to the patient.

It is noted that in some embodiments of this preferred embodiment, when the system 100 comprises more than one sensor 20, at least one sensor 20 is comprised within a mechanical ventilation system, while the other sensors 20 may be not comprised within the mechanical ventilation system.

In another preferred embodiment according to any one of the embodiments of the first aspect of the invention, the actuation of the TERM system, such as the application of the at least two electrical stimuli, is further adapted based on mechanical ventilated-related variables, preferably wherein the mechanical ventilated-related variables comprise any of: airway pressure (Paw), flow, and volume pressure. These mechanical ventilated-related variables enable proper synchronization with the breathing state of the patient, as the breathing state can be inferred from the readings of one or more of these variables. The TERM system may be actuated for example through one or more multifield skin electrodes (11a, 11b).

In another preferred embodiment according to any one of the embodiments of the first aspect of the invention, the biometric measurement is an electrocardiogram (ECG) and the monitoring device an electrocardiogram monitoring device.

As shown in Example 1, the system for synchronized respiratory muscular electrical stimulation of a patient according to any one of the embodiments of the first aspect of the invention can effectively stimulate the respiration muscles of the patient without affecting the ECG measurements such that they can still be used by the medical staff and controlled by automatic monitoring systems.

In another preferred embodiment according to any one of the embodiments of the first aspect of the invention, the system for synchronized respiratory muscular electrical stimulation of a patient, further comprises at least one electromyography (EMG) sensor, configured to measure the activity of one or more of the respiratory muscles of the patient.

Moreover, in this preferred embodiment the processor (30) is further configured to actuate the TERM system (10) considering the activity of one or more of the respiratory muscles of the patient as measured by the EMG sensor.

More preferably, the processor is further configured to determine the fatigue of the respiratory muscles of the patient from the activity registered by the at least one EMG sensor and to actuate the RMES system considering the fatigue status of the respiratory muscles of the patient.

An EMG sensor is a sensor configured to register the electrical activity of the muscles of the patient. It is noted that the EMG sensor may be of different types including bipolar electrodes.

A system comprising at least one EMG sensor can further use the activity of one or more of the respiratory muscles of the patient measured by the EMG sensor to modulate the actuation of the TERM system, such as the at least two electrical stimuli provided by the TERM system. This in turn can be used to infer different properties of the patient, such as the muscle fatigue, respiratory cycles, maximum contraction of the muscle, and/or the contraction speed of muscle fibres which can be used to adapt the stimuli properties such as amplitude and/or periods.

It is noted that from the above disclosure, the skilled person may envisage many different strategies the TERM system may be configured to ensure muscle fatigue is under control. For example, if muscle fatigue is detected, it can be stablished that a resting period of a certain amount of time, such as a breathing cycle shall be stablished until the fatigue is diminished or until a predetermined time threshold is reached. Alternatively, or additionally, the amplitude of the stimulation can be diminished to reduce the fatigue produced by new stimulations.

The skilled person may envisage many different strategies the TERM system may be configured to ensure muscle fatigue is under control such as alternating different series of stimuli with series of resting. Likewise, series of high intensity electrical stimuli may be alternated with series of low intensity electrical stimuli. These may be even combined. All of these different possible combinations of period and intensity variations that may be envisaged from the above are also comprised within the first aspect of the invention.

Advantageously, the system for synchronized respiratory muscular electrical stimulation of a patient according to this preferred embodiment can effectively ensure the electrical stimulation is being provided to the respiratory muscles, while fatigue of the same muscles is prevented.

In another preferred embodiment according to any one of the embodiments of the first aspect of the invention, the TERM system comprises at least one calibratable element, and the processor (30) is further configured to actuate the TERM system (10) in a predetermined way according to a previous calibration.

Advantageously, a system (100) according to the first aspect of the invention wherein the TERM system comprises at least one calibratable element, and wherein the processor (30) is further configured to actuate the TERM system (10) in a predetermined way according to a previous calibration enables the tailoring of the synchronized respiratory muscular electrical stimulation of the patient according to his/her physiology, medical conditions, medical treatments and/or environment.

In another preferred embodiment according to any one of the embodiments of the first aspect of the invention, each of the one or more skin electrodes (11a, 11b) are multifield electrodes comprising an anode and a cathode.

A multifield electrode is a type of electrode that includes multiple, distinct regions or fields within a single electrode unit. These fields can be selectively activated to allow precise targeting of specific muscle groups or areas, to provide greater control in electrical stimulation therapy. The multifield configuration enhances the flexibility of the stimulation patterns, enabling more effective and focused treatment for respiratory muscle stimulation.

A multifield electrode may comprise different fields for the anode and the cathode as can be seen in Fig. 6A and 6B. This means that within the same electrode unit, there can be separate designated fields that function as the anode and the cathode. The spatial arrangement of these fields allows for the generation of electrical currents that can be directed more accurately to stimulate the desired muscle regions. By having separate fields for the anode and cathode, the system can optimize the flow of electrical impulses, thereby improving the efficacy of the stimulation therapy. It is noted that although the multifield electrodes in Fig. 6A and 6B have a particular field disposition, field shapes and field sizes, the first aspect of the invention is not necessarily limited whatsoever to a particular shape or relative arrangement of the fields, nor to a particular field size. It is also noted that several fields may defined a bigger field, and therefore different sizes of fields may be defined by combining adjacent fields. Therefore, a skilled person can envisage many other alternatives to the multifield electrodes disclosed in Figs. 6A and 6B, all of which are comprised within the first aspect of the invention.

Furthermore, it should be noted that only some or even just one of the fields of the said electrode may be activated at any given time. Activating only a subset of fields allows the electrical stimulation to be focused on a particular location, minimizing unwanted stimulation of adjacent tissues which could lead to patient discomfort or signal interference with a further monitoring device.

In a more preferred embodiment, the processor (30) is further configured to actuate the one or more multifield skin electrodes (11a, 11b) at a current intensity of at least 1mA, preferably, 2, 3, 4, 5, 6, 7, 8, 9, 10, 11, 12, 13, 14, 15, 16, 17, 18, 19 more preferably 20 mA.

Advantageously, a current intensity of at least 20 mA enables the superficial stimulation of respiratory muscles directly through the stimulation of their motor points of the muscle, even in muscles further from the skin surface, such as the diaphragm.

Notably this current intensity differs from the stimulation of muscles indirectly through nerve stimulation, since nerves require focused, less intense currents to ensure only the desired muscles are activated. Providing a current intensity of 20 mA or more towards such a nerve would not only stimulate surrounding structures such as muscles and other nerves, but would also lead to pain into the patient.

As shown in Example 5, the TERM stimulation with a current intensity of at least 20 mA, can successfully activate the diaphragm muscle.

It is further noted, as explained above, that a current intensity of at least 20mA can distort one or more biometric measurements performed by one or more monitoring devices on the patient.

In another more preferred embodiment, the processor (30) is further configured to actuate the one or more multifield skin electrodes (11a, 11b) at a current intensity comprised between 20 mA and 100 mA. As shown in examples 5 and 6, the TERM stimulation comprised between 20 mA and 100 mA, can successfully activate the diaphragm muscle.

In a further preferred embodiment, when the processor is configured to actuate the TERM system when the breathing state is in the inspiration phase the processor is further configured to actuate the one or more multifield skin electrodes at a current intensity comprised between 20 mA and 60 mA. Advantageously, this current intensity is further tailored for inspiration muscles. More preferably, the processor is further configured to actuate the one or more multifield skin electrodes at a current intensity comprised between 20 mA and 59, 58, 57, 56, 55, 54, 53, 52, 51 mA, further preferably between 20mA and 50 mA.

In another further preferred embodiment, when the processor is configured to actuate the TERM system when the breathing state is in the expiration phase the processor is further configured to actuate the one or more multifield skin electrodes at a current intensity comprised between 15 mA and 100 mA. More preferably, the processor is further configured to actuate the one or more multifield skin electrodes at a current intensity comprised between 16, 17, 18, 19, 20 , 21, 22, 23, 24, 25, 26, 27, 28, 29, 30, 31, 32, 33, 34, 35, 36, 37, 38, 39 mA, further preferably between 40 mA and 100 mA. Advantageously, this current intensity is further tailored for expiration muscles.

More preferably, the processor is further configured to actuate the one or more multifield skin electrodes at a current intensity comprised between 40 mA and 99, 98, 97, 96, 95, 94, 93, 92, 91, 90 mA.

In a more preferred embodiment, the processor (30) is further configured to actuate the one or more multifield skin electrodes (11a, 11b) at a predetermined field and a current within a predetermined current range.

The term 'predetermined field' refers to a specific field of the multifield electrode that has been selected beforehand for activation, as will be now described. Similarly, the term 'predetermined current range' refers to a certain current range that has been selected in advance according to the patient's therapeutic needs and tolerance levels. This current range defines a set of values within a minimum and maximum current levels that can be applied to the selected field of the electrode as will be now described. By operating within this predetermined current range, the system ensures safe, controlled, and effective stimulation tailored to the patient's condition without interfering or distorting a signal from a further monitoring device.

In this more preferred embodiment, the predetermined field as well as the minimum of said predetermined current range has been predetermined according to a particular set of steps as will be now disclosed.

It is noted that although only the minimum of said predetermined current range is provided, the skilled person can still derive a current within such predetermined current range. For instance, the current within a predetermined current range wherein only the minimum of said current range has been provided may be such minimum value.

In this more preferred embodiment, the predetermined field as well as the minimum of said predetermined current range has been predetermined according to the following steps:
a. Stimulating a first cathode field of the electrode at a minimum current. The minimum current is predetermined by the TERM system. It is noted that the minimum current may be the minimum current the TERM system may be able to provide, or a minimum current value predefined by the TERM system or set by a technician into the TERM system according to the patient physiognomy. For example, the minimum current may be just over 0mA.
   The first cathode field may be a random cathode field or a predefined cathode field. For example, the first cathode field may be the field of the multifield electrode that, upon placement over the respiratory muscles of the patient, is placed between the 8th and 10th intercostal space at the level of the mid-axillary line. The skilled person may envisage many other alternatives in which the skilled person may predefine a cathode field to be the first cathode field, for example according to its relative location to other cathode fields or to a specific anatomical position upon placement over the respiratory muscles of the patient.
b. Increasing the current of step a until there is a detectable respiratory muscle contraction. This increase in current may be continuous or discrete and different speeds in the increase of current may be used. It is noted that the respiratory muscle contraction may be detectable through many different means as the skilled person may envisaged. For example, the respiratory muscle contraction may be detectable through an accelerometer, a respiratory band an electromyograph electrode or the same. Alternatively, a computer-vision system may determine the contraction through imagen recognition or an additional subject may provide such indication. All of the above as well as any other alternative the skilled person may envisage are deemed to be comprised within the present embodiment.
c. Associating the current of step (b) to the cathode field of step (a).
d. Repeating steps (a) to (c) for at least an additional cathode field of the one or more multifield skin electrodes (11a, 11b). Therefore, for each cathode field of the one or more multifield skin electrode the minimum current which results in a muscle contraction is associated to such cathode field.
e. Determining the cathode field with the minimum current required to generate a detectable respiratory muscle contraction as the predetermined field, and determining the current required to generate a detectable respiratory muscle contraction in said predetermined field as the minimum of the predetermined current range.

Advantageously, a system according to this preferred embodiment ensures an effective stimulation of the respiratory muscles of the patient that does not distort one or more biometric measurements performed by one or more monitoring devices as shown in Example 4.

In another preferred embodiment according to any one of the embodiments of the first aspect of the invention, the processor is configured to actuate the TERM system when the breathing state is in the inspiration phase.

In another more preferred embodiment, the step (d) comprises repeating steps (a) to (c) for at least an additional 1, 2, 3, 4, 5, 6, 7 8, 9, 10, 11, 12, 13, 14, 15, 16, 17, 18, 19, 20, 25, 30, 35, 40 or 50 cathode fields of the one or more multifield skin electrodes (11a, 11b), preferably wherein the additional 1, 2, 3, 4, 5, 6, 7 8, 9, 10, 11, 12, 13, 14, 15, 16, 17, 18, 19, 20, 25, 30, 35, 40 or 50 cathode fields are selected according to the proximity to the first cathode field.

In another more preferred embodiment, the step (d) comprises repeating steps (a) to (c) for each of the cathode fields of the one or more multifield skin electrodes (11a, 11b).

In another more preferred embodiment, at least one of the one or more skin electrodes (11a, 11b) (11a, 11b) comprises a positioning marker (61) configured to guide the placement of the at least one of the one or more skin electrodes (11a, 11b) over the respiratory muscles of the patient. More preferably, each of the one or more skin electrodes (11a, 11b) (11a, 11b) comprises a positioning marker (61) configured to guide the placement over the respiratory muscles of the patient.

The positioning marker (61) may be any visual or haptic element configured to indicate that a certain part of the multifield electrode is configured to be placed onto a certain anatomical part of the body of the patient, such as the xiphoid process or the anterior superior iliac spine. For example, for the one or more skin electrodes (11a, 11b) configured to be placed over the inspiration respiratory muscles, as shown in in Fig. 6A the multifield electrode may comprise a positioning marker (61) between both anodes, to indicate that, when in use, such location should be placed over the xiphoid process. Likewise, for the one or more skin electrodes (11a, 11b) configured to be placed over the expiratory respiratory muscles as shown in in Fig. 6B, the multifield electrode may comprise a positioning marker (61) between each anode and cathode, to indicate that, when in use, such location should be placed over the anterior superior iliac spine In this way, the positioning marker (61) is configured to guide the placement over the respiratory muscles of the patient. The skilled person may envisage many types of positioning markers such as shapes, text, drawings, textures, and/or colours that may be used to indicate a certain part of the multifield electrode is configured to be placed onto a certain anatomical part of the body of the patient, all of which are comprised within this embodiment.

In another more preferred embodiment, when the processor is configured to actuate the TERM system when the breathing state is in the inspiration phase, at least one, preferably each of the one or more skin electrodes (11a, 11b) comprises a positioning marker configured to guide the placement of the at least one of the one or more skin electrodes (11a, 11b) over the respiratory muscles of the patient, and the first cathode field is the field of the multifield electrode positioned within the multifield electrode set between the 8^{th} and 10^{th} intercostal space at the level of the mid-axillary line with respect to the placement position of the positioning marker.

Advantageously, the area between the 8^{th} and 10^{th} intercostal space at the level of the mid-axillary line is the area which requires the minimum current to produce a respiratory muscle contraction, so by selecting the field of the multifield electrode located with respect to the mark such that, upon placement over the respiratory muscles of the patient, is placed between the 8^{th} and 10^{th} intercostal space at the level of the mid-axillary line as the first cathode field increases the possibility of finding the predetermined fields in the first measurements.

In another more preferred embodiment, the maximum of said predetermined current range has been predetermined by the processor according to the following steps:
f. Stimulating the predetermined field determined in step (a) at the minimum of the predetermined current range as defined in step (e).
g. Increasing the current of step (f) until the detectable respiratory muscle contraction does not increase further. This increase in current may be continuous or discrete and different speeds in the increase of current may be used. The respiratory muscle contraction may be recorded using different means, such as an accelerometer, a respiratory band an electromyograph electrode or the same. The skilled person may envisage many alternatives in which the respiratory muscle contraction may be determined.
h. Finally, determining the minimum intensity that provides the maximum detectable respiratory muscle contraction in said predetermined field as the maximum of the predetermined current range. Since the minimum intensity that provides the maximum detectable respiratory muscle contraction is the least amount of current required to achieve the maximum detectable respiratory muscle contraction for such patient, it can be selected as the maximum of the predetermined current range.

Advantageously, the system is further configured to provide a maximum value for the predetermined current range that ensures maximum effectivity on the maximum of the respiratory muscle contraction at the least current, minimising the risk of distorting one or more biometric measurements performed by one or more monitoring devices. Moreover, in combination with the minimum value, it defines a current range of effective respiratory muscle contraction with the least distortion of one or more biometric measurements performed by one or more monitoring devices.

In another more preferred embodiment, the steps (a) to (d) for the predetermined field and the minimum of the predetermined current range have been performed using a minimised cathode field, wherein the minimised cathode field is a cathode field smaller than the anode field. Therefore, during the (a) to (d) steps of finding the predetermined field and the minimum of the predetermined current range, the field of the cathode activated has a smaller area than the field of the anode activated. Advantageously, since current density is inversely correlated with the area, the minimised cathode field has an increased current density while the resolution of the cathode with respect of the respiratory muscle is increased, allowing to find the best area,

Then, according to this more preferred embodiment, the steps for determining the predetermined field and the minimum of said predetermined current range further comprises, after step (d) and before step (e):
d1. Selecting the cathode field with the minimum current required to generate a detectable respiratory muscle contraction as a minimised cathode field. In this step, from all the cathode fields the one which requires the least current to generate a detectable respiratory muscle contraction is selected as a minimised cathode field. The term "minimised cathode field" refers to a cathode field that has a smaller area than the field of the anode activated.
d2. Increasing the area of the cathode field to be the same as the area of the anode field.
d3. Repeating steps (a) to (c) for each cathode field of the one or more multifield skin electrodes (11a, 11b) comprising the minimised cathode field of step (d1).

Advantageously, the predetermined area selected at step (e) comprises the minimised cathode area, i.e. an area with smaller resolution, which requires the least current to generate a detectable respiratory muscle contraction.

In another preferred embodiment according to any one of the embodiments of the first aspect of the invention,, the processor is configured to actuate the TERM system when the breathing state is in the inspiration phase and in expiration phase.

In another more preferred embodiment, when the processor is configured to actuate the TERM system when the breathing state is in the expiration phase, the predetermined current range has been predetermined according to the following steps:
a. stimulating a cathode field of the electrode at a minimum current, wherein the minimum current is the minimum current as determined by the TERM system;
b. increasing the current of step (a) until a detectable respiratory muscle contraction does not increase further; and
c. determining the minimum intensity that provides a detectable respiratory muscle contraction does not increase further as the maximum of the predetermined current range.

In another more preferred embodiment, the TERM system is configured to actuate the one or more multifield skin electrodes (11a, 11b) using an anode field and cathode field with an area between 10 and 50 cm², preferably 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 cm². Advantageously, an area between 10 and 50 cm², preferably 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 cm2 provides an appropriate area for detectable muscular stimulation without providing pain to the patient.

In another more preferred embodiment, the maximum of the predetermined current range is determined as a factor of the minimum of the predetermined current range, wherein the factor is selected according to the body mass index of the patient.

Advantageously, the maximum of the predetermined current range can be determined in a faster way, reducing the time to predetermine the current range.

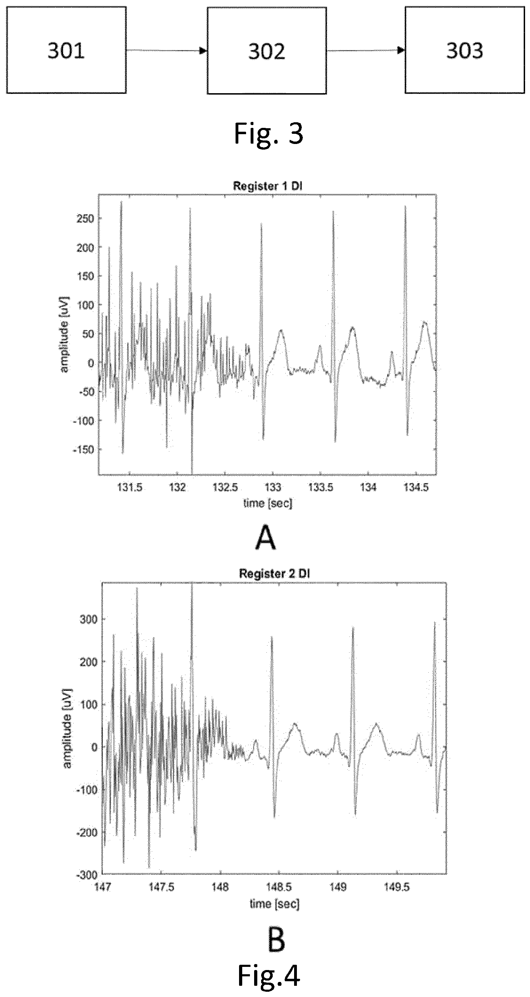

A second aspect of the invention relates to a method for synchronized electrical stimulation with a system 100 for synchronized respiratory muscular electrical stimulation of a patient according to any one of the embodiments of the first aspect of the invention. As shown with respect to Fig. 3 the method comprises the following steps.

In a first step 301 receiving information regarding the breathing state of the patient preferably from a sensor 20 connected to a mechanical ventilation system. It is noted that the breathing state may be received directly as state, i.e. either an inspiratory state or expiratory state, or indirectly in the form of a signal or set of values from wherein the breathing state can be computed. For example, an air flow measurement, wherein the flow is depicted as positive if inserted into the patient and negative if extracted from the patient, can be received. From such signal, it can be computed that when the signal is positive the breathing state is the inspiration state and when the signal is negative the breathing state is the expiration state.

In a second step 302 the method involves determining from the information if the breathing state corresponds to a selected breathing phase of the patient. Thus, in this step the received breathing state is compared to a selected breathing phase of the patient of interest, which can be the inspiration phase and/or the expiration phase.

In a third step 303, the method involves, when the breathing state corresponds to the selected breathing phase of the patient, indicating to the processor 30 that the breathing state corresponds to the selected breathing phase of the patient.

Advantageously, given that the system 100 for synchronized respiratory muscular electrical stimulation of a patient according to any one of the embodiments of the first aspect of the invention comprises an TERM system 10 and a processor 30, and the processor 30 is configured to actuate the TERM system 10 when the breathing state is in the inspiration phase and/or the expiration phase, when the processor 30 is indicated that the breathing state corresponds to the selected breathing phase of the patient, at least two electrical stimuli with an intensity that distorts the one or more biometric measurements performed by one or more monitoring devices and with a period of at least 200 milliseconds is provided to the respiratory muscles of the patient. Thus, the method according to the second aspect of the invention ensures the synchronized electrical stimulation with a system 100 for synchronized respiratory muscular electrical stimulation of a patient according to any one of the embodiments of the first aspect of the invention.

In a preferred embodiment, the method according to the second aspect of the invention is a computer implemented method.

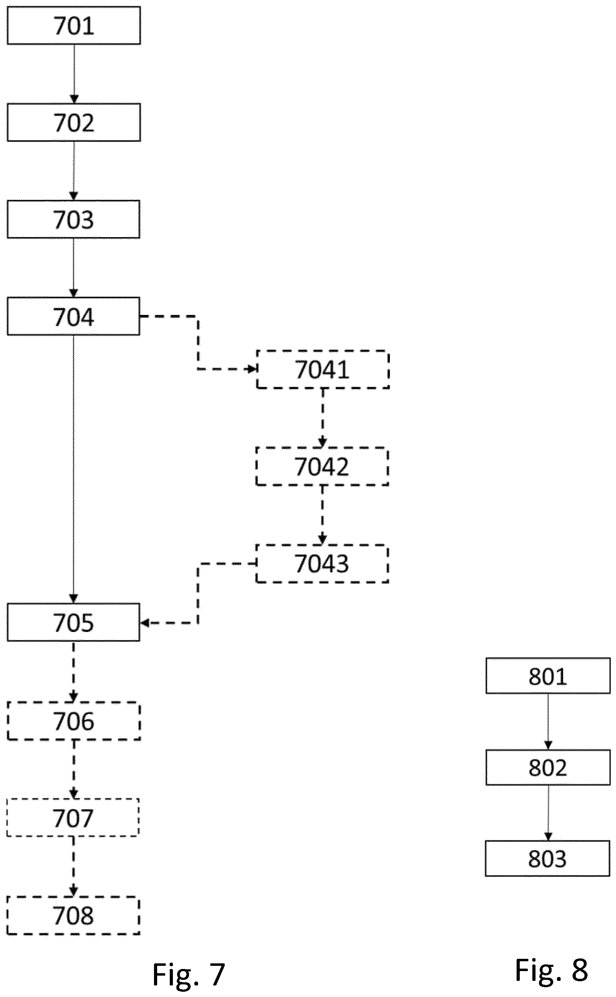

A third aspect of the invention refers to a computer implemented method for determining the field and current range to actuate the one or more multifield skin electrodes (11a, 11b) of a system (100) for synchronized respiratory muscular electrical stimulation of a patient according to any one of the embodiments of the first aspect of the invention. As shown with respect to Fig. 7 the method comprises the following steps.

In a first step 701, stimulating a first cathode field of the electrode at a minimum current, the minimum current being the minimum current as determined by the TERM system. It is noted that the minimum current may be the minimum current the TERM system may be able to provide, or a minimum current value predefined by the TERM system or set by a technician into the TERM system according to the patient physiognomy. The first cathode field may be a random cathode field or a predefined cathode field. For example, the first cathode field may be the field of the multifield electrode that, upon placement over the respiratory muscles of the patient, is placed between the 8th and 10th intercostal space at the level of the mid-axillary line.

In a second step 702 the method involves increasing the current of step 701 until there is a detectable respiratory muscle contraction. This increase in current may be . It is noted that the respiratory muscle contraction may be detectable through many different means as the skilled person may envisaged. For example, the respiratory muscle contraction may be detectable through an accelerometer, a respiratory band an electromyograph electrode or the same. Alternatively, a computer-vision system may determine the contraction through imagen recognition or an additional subject may provide such indication. All of the above as well as any other alternative the skilled person may envisage are deemed to be comprised within the present embodiment.

In a third step 703, the method involves associating the current of step 702 to the cathode field of step 701.

A fourth step 704 of the method involves repeating steps 701 to 703 for at least an additional cathode field of the one or more multifield skin electrodes (11a, 11b). Therefore, for each cathode field of the one or more multifield skin electrode the minimum current which results in a muscle contraction is associated to such cathode field.

Finally, step 705 of the method involves determining the cathode field with the minimum current required to generate a detectable respiratory muscle contraction as the predetermined field, and determining the current required to generate a detectable respiratory muscle contraction in said predetermined field as the minimum of the predetermined current range.

Advantageously, this method ensures that the field and current range to actuate the one or more multifield skin electrodes (11a, 11b) of a system (100) for synchronized respiratory muscular electrical stimulation of a patient according to any one of the embodiments of the first aspect of the invention are determined considering the least current needed to generate a respiratory muscle contraction. In turn this means that the stimulation provided by the TERM system (10) of said system (100) for synchronized respiratory muscular electrical stimulation is done without interfering or distorting a signal from a further monitoring device or interferes as little as possible with it. Thus, the method according to the third aspect of the invention ensures the electrical stimulation with a system 100 for synchronized respiratory muscular electrical stimulation of a patient according to any one of the embodiments of the first, aspect of the invention is as little intrusive as possible for any other monitoring device monitoring the patient. It is noted that in the embodiments of the first aspect of the invention where patients require breathing assistance, they are usually continuously monitored, therefore it is essential that the TERM stimulation does not prevent such monitorization.

It is noted that in Fig. 7, there are multiple dashed steps, which are optional steps as will be described now with respect to preferred aspects of the third aspect of the invention.

In a preferred embodiment of the third aspect of the invention, the step 704 comprises repeating steps 701 to 703 for at least an additional 1, 2, 3, 4, 5, 6, 7 8, 9, or 10 cathode fields of the one or more multifield skin electrodes (11a, 11b), preferably wherein the additional 1, 2, 3, 4, 5, 6, 7 8, 9, or 10 cathode fields are selected according to the proximity to the first cathode field.

In another preferred embodiment of the third aspect of the invention, the step (d) comprises repeating steps (a) to (c) for each of the cathode fields of the one or more multifield skin electrodes (11a, 11b).

In another preferred embodiment of the third aspect of the invention, as shown with respect to Fig. 7 the method further comprises the following steps:
In a sixth step 706 stimulating the predetermined field determined in step 705 at the minimum of the predetermined current range as defined in step 705.

Then, in a seventh step 707, increasing the current of step 706 until the detectable respiratory muscle contraction does not increase further. This increase in current may be continuous or discrete and different speeds in the increase of current may be used. The respiratory muscle contraction may be recorded using different means, such as an accelerometer, a respiratory band an electromyograph electrode or the same. The skilled person may envisage many alternatives in which the respiratory muscle contraction may be determined.

Finally, in the step 708 determining the minimum intensity that provides the maximum detectable respiratory muscle contraction in said predetermined field as the maximum of the predetermined current range. Since the minimum intensity that provides the maximum detectable respiratory muscle contraction is the least amount of current required to achieve the maximum respiratory muscle contraction for such patient, it can be selected as the maximum of the predetermined current range.

Advantageously, the method of the third aspect of the invention ensures that the current range to actuate the one or more multifield skin electrodes (11a, 11b) of a system (100) for synchronized respiratory muscular electrical stimulation of a patient according to any one of the embodiments of the first, aspect of the invention has a maximum value for the predetermined current range that ensures maximum effectivity on the maximum of the respiratory muscle contraction at the least current, minimising the risk of distorting one or more biometric measurements performed by one or more monitoring devices. Moreover, in combination with the minimum value, it provides a current range of effective respiratory muscle contraction with the least distortion of one or more biometric measurements performed by one or more monitoring devices.

In another preferred embodiment of the third aspect of the invention as shown with respect to Fig. 7, the steps 701 to 704 for the predetermined area and the minimum of the predetermined current range have been performed using a minimised cathode field, wherein the minimised cathode field is a cathode field smaller than the anode field and the method further comprises after step 704 and before step 705 the following steps:
According to step 7041 selecting the cathode field with the minimum current required to generate a detectable respiratory muscle contraction as a minimised cathode field. In this step, from all the cathode fields the one which requires the least current to generate a detectable respiratory muscle contraction is selected as a minimised cathode field. The term "minimised cathode field" refers to a cathode field that has a smaller area than the field of the anode activated.

Then in step 7042 increasing the area of the cathode field to be the same as the area of the anode field.

Finally in step 7043 repeating steps 701 to 703 for each cathode field of the one or more multifield skin electrodes (11a, 11b) comprising the minimised cathode field of step 7041.

Advantageously, the predetermined area selected at step 705 comprises the minimised cathode area, i.e. an area with smaller resolution, which requires the least current to generate a detectable respiratory muscle contraction.

In another preferred embodiment of the third aspect of the invention and as shown in Fig. 8, when the processor is configured to actuate the TERM system when the breathing state is in the expiration phase, the predetermined current range has been predetermined according to the following steps:
In a first step 801, stimulating a cathode field of the electrode at a minimum current, the minimum current being the minimum current as determined by the TERM system. It is noted that the minimum current may be the minimum current the TERM system may be able to provide, or a minimum current value predefined by the TERM system or set by a technician into the TERM system according to the patient physiognomy. The first cathode field may be a random cathode field or a predefined cathode field. For example, the first cathode field may be the field of the multifield electrode that, upon placement over the respiratory muscles of the patient, is placed between the 8th and 10th intercostal space at the level of the mid-axillary line.

Then, in a second step 802, increasing the current of step 801 until the detectable respiratory muscle contraction does not increase further. This increase in current may be continuous or discrete and different speeds in the increase of current may be used. The respiratory muscle contraction may be recorded using different means, such as an accelerometer, a respiratory band an electromyograph electrode or the same. The skilled person may envisage many alternatives in which the respiratory muscle contraction may be determined.

Finally, in the step 803 determining the minimum intensity that provides the maximum detectable respiratory muscle contraction in said predetermined field as the maximum of the predetermined current range. Since the minimum intensity that provides the maximum detectable respiratory muscle contraction is the least amount of current required to achieve the maximum detectable respiratory muscle contraction for such patient, it can be selected as the maximum of the predetermined current range.

A fourth aspect of the invention refers to a computer program product comprising instructions configured to perform the method according to the second and/or third aspect of the invention.

All of the above are fully within the scope of the present disclosure and are considered to form the basis for alternative embodiments in which one or more combinations of the above-described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

### Examples

### Example 1: Impact of TERM on ECG monitoring

During the stimulation therapy, we have observed that the current applied by the device distorts the measurement of biomedical signals recorded in the ICU. One of the crucial signals affected is the ECG, which requires constant monitoring.

The objective was to determine whether TERM (transcutaneous electrostimulation of the respiratory muscles) interferes with ECG monitoring, considering:
1) The position of the electrodes
2) The intensity of the current

### Methodology

### Position 1: INSPIRATION - LATERAL

1. The electrodes for electrostimulation shall be placed in the position of inspiration - lateral:
   - Two electrodes in the 7th intercostal space in the midaxillary line.
   - Two electrodes in the paraxiphoid region.
2. Electrostimulation will start at 0 mA, and will increase in values of 5 mA.
3. For each intensity value, the following shall be recorded:
   a) If there is a distortion in the monitoring of the ECG;
   b) Whether the distortion is permissible;
   c) If the distortion triggers a monitoring alarm;
   d) If the distortion disappears at the moment the electrostimulation stops, or if, on the contrary, it is maintained for a few seconds.

### Position 2: INSPIRATION - BACK

1. The electrodes for electrostimulation shall be placed in the posterior inhalation position:
2. Electrostimulation will start at 0 mA, and will increase in values of 5 mA.
3. For each intensity value, the following shall be recorded:
   a) If there is a distortion in the monitoring of the ECG;
   b) Whether the distortion is permissible;
   c) If the distortion triggers a monitoring alarm;
   d) If the distortion disappears at the moment the electrostimulation stops, or if, on the contrary, it is maintained for a few seconds.

### Position 3: EXPIRATION

1. The electrodes for electrostimulation shall be placed in the expired position:
   - Two electrodes (2 and 3) 2 cm below and parallel to the costal margin from the line medium, inclined diagonally downwards in an inferolateral direction, towards the anterosuperior iliac spine.
   - Two electrodes (1 and 4) directed from the middle axillary line in the eighth rib obliquely towards the posterolateral aspect of the iliac crest, covering the two inferior intercostal spaces, the posterolateral abdominal wall and many of the nerves that innervate the abdominal muscles.
2. Electrostimulation will start at 0 mA, and will increase in values of 5 mA.
3. For each intensity value, the following shall be recorded:
   a) If there is a distortion in the monitoring of the ECG;
   b) Whether the distortion is permissible;
   c) If the distortion triggers a monitoring alarm;
   d) If the distortion disappears at the moment the electrostimulation stops, or if, on the contrary, it is maintained for a few seconds.

### Results

| **Position** | **Intensity [mA]** | **ECG distortion** | **Distortion acceptable** | **ECG alarm** | **Distortion disappears immediately** |
|---|---|---|---|---|---|
| Inspiration - lateral | 5 | Yes | Yes | No | Yes |
| | 10 | Yes | Yes | No | Yes |
| | 15 | Yes | No | Yes | Yes |
| Inspiration - posterior | 5 | Yes | Yes | No | Yes |
| | 10 | Yes | Yes | No | Yes |
| | 15 | Yes | Yes | No | Yes |
| | 20 | Yes | Yes | No | Yes |
| | 25 | Yes | No | No | Yes |
| | 30 | Yes | No | No | Yes |
| Expiration | 5 | Yes | Yes | No | Yes |
| | 10 | Yes | Yes | No | Yes |
| | 15 | Yes | No | Yes | Yes |

### Conclusions

In this first example, it can be concluded the following facts:
- The ECG becomes distorted even when stimulating at low intensities, such as 5 mA. As will be explained later with respect to Example 3, to achieve an effect on the diaphragm, we need to stimulate with intensities of at least around 20 mA. Therefore, intensity is not an adjustable parameter to avoid ECG distortion.
- The distortion becomes unacceptable starting at 15 mA during lateral inspiration and expiration and starting at 30 mA during posterior inspiration. Since neither of these intensity values is suitable for stimulation across all individuals, the electrode placement is also not adjustable.
- In the lateral inspiration and expiration positions, the alarm triggers when the electrical stimuli has an intensity of at least 15 mA.
- It is observed that the ECG almost immediately returns to its normal state once the stimulation is discontinued.

### Example 2: Impact of different stimulation frequencies on resting periods on ECG monitoring

We aimed to obtain ECG signals with interference from electrical stimulation at 30 and 70 Hz and then attempt to filter them. We analysed recovery times and examined whether there are differences between stimulating at 30 or 70 Hz.

However, the ECG is not the unique signal affected by the application of electrical stimulation currents. Other biomedical signals, such as EMG (Electromyography) and EEG (Electroencephalography), also experience this interference:
- EMG: This is used in the ICU to record muscle fatigue in patients (as in our case). It is employed during sedation processes to assess the patient's voluntary muscle activity and to analyse neuromuscular transmission in patients with neuromuscular pathologies.
- EEG: This provides information on the patient's neuronal activity, making it valuable for diagnosing epilepsy, detecting cerebral ischemia, monitoring sedation depth, and assessing conditions like encephalopathy or encephalitis.

### Methodology

The first and second derivations (D. I and D. II, respectively) were measured. Four different records were made.

### Record 1

Place the ECG recording electrodes as in routine practice in ICU mechanical ventilation. Place the ECG stimulation electrodes in the inspiration position.
- Two electrodes in the 7th intercostal space in the midaxillary line.
- Two electrodes in the paraxiphoid region.

Stimulate with the following parameters:
- Sufficient intensity (that does not cause pain but that the stimulation is noticed visually or that the subject notices it).
- Stimulation frequency 30 Hz
- Pulse duration 350 us
- Working time: 4 seconds; Rest time: 5 seconds

Record for 3 minutes = 180 seconds. 2 minutes with 15mA and 1 minute with 20mA

### Record 2

Place the electrodes recording ECG as in routine practice in ICU mechanical ventilation. Place the ECG stimulation electrodes in the inspiration position.
- Two electrodes in the 7th intercostal space in the midaxillary line.
- Two electrodes in the paraxiphoid region.

Stimulate with the following parameters:
- Sufficient intensity (that does not cause pain but that the stimulation is noticed visually or that the subject notices it).
- Stimulation frequency 70 Hz
- Pulse duration 350 us
- Working time: 4 seconds; Rest time: 5 seconds

Record for 3 minutes = 180 seconds. 1 minute with 15mA; 1 minute with 20 mA; 1 minute with 23Ma.

### Record 3

Place the electrodes recording ECG as in routine practice in ICU mechanical ventilation. Place the ECG stimulation electrodes in the expiration position.

Stimulate with the following parameters:
- 1.Sufficient intensity (that does not cause pain but that the stimulation is noticed visually or that the subject notices it).
- 2.Stimulation frequency 30Hz
- 3.Pulse duration 350 us
- 4.Working time: 4 seconds; Rest time: 5 seconds

Record for 3 minutes = 180 seconds: 30 secs at 9mA, 30 secs at 15mA, 30 secs at 20mA, 1 min at 25mA, 30 secs at 27mA.

### Record 4

Place the electrodes recording ECG as in routine practice in ICU mechanical ventilation. Place the ECG stimulation electrodes in the expiration position.

Stimulate with the following parameters:
- Sufficient intensity (that does not cause pain but that the stimulation is noticed visually or that the subject notices it).
- Stimulation frequency 70 Hz
- Pulse duration 350 us
- Working time: 4 seconds; Rest time: 5 seconds

Record for 3 minutes = 180 seconds: 30 secs at 9mA, 30 secs at 15mA, 1 min at 18mA, 30 secs at 20mA, 30 secs at 25mA.

For each of these recordings, the ECG signal recovery was measured using both manually and with a spectrogram analysis.

The spectrogram analysis was performed as follows:
Obtention of the spectrogram with Hamming window size 60 samples and overlay 20 samples.
1. Calculation of signal energy with windows of 20 samples to have measurements of the order of 0.1 seconds (the minimum observable). Calculation from 40 hertz
2. Calculation of the time between the last maximum peak of each period and the next threshold using a threshold for detecting peaks of 450 energy units and threshold for consider it to be ECG information of 100 energy units.

### Results

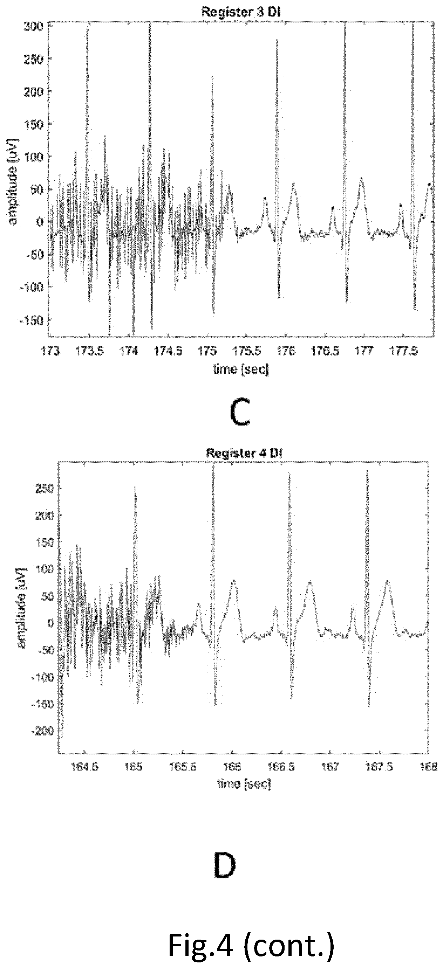

A detailed capture of the first derivation of register 1 at 20mA can be seen in Fig. 4A, a detailed capture of the first derivation of register 2 at 23mA can be seen in Fig. 4B, a detailed capture of the first derivation of register 3 at 27mA can be seen in Fig. 4C and a detailed capture of the first derivation of register 4 at 23mA can be seen in Fig. 4D.

The resting period needed to recover a clinically useful ECG signal was the following:

Manual method (measured in seconds):

| **Inspiration lead I** | | | | |
|---|---|---|---|---|
| | 0-10 mA | 10-15 mA | 15-20 mA | 20-25 mA |
| 30 Hz (R1) | - | 0,385 | 0,575 | - |
| 70 Hz (R2) | - | 0,410 | 0,325 | 0,335 |

| **Inspiration lead II** | | | | |
|---|---|---|---|---|
| | 0-10 mA | 10-15 mA | 15-20 mA | 20-25 mA |
| 30 Hz (R1) | - | 0,5 | 0,57 | - |
| 70 Hz (R2) | - | 0,46 | 0,435 | 0,42 |

| **Expiration lead I** | | | | | |
|---|---|---|---|---|---|
| | 0-10 mA | 10-15 mA | 15-20 mA | 20-25 mA | 27 mA |
| 30 Hz (R3) | 0,4356 | 0,585 | 0,4 | 0,384 | 0,425 |
| 70 Hz (R5) | 0,615 | 0,3401 | 0,47 | 0,385 | - |

| **Expiration lead II** | | | | | |
|---|---|---|---|---|---|
| | 0-10 mA | 10-15 mA | 15-20 mA | 20-25 mA | 27 mA |
| 30 Hz (R3) | 0,485 | 0,54 | 0,575 | 0,395 | 0,36 |
| 70 Hz (R5) | 0,41 | 0,4301 | 0,555 | 0,44 | - |

| Minimum value | Maximum value | Mean |
|---|---|---|
| 0,325 | 0,615 | 0,4516 |

Spectrogram method (measured in seconds):

| **Inspiration lead I** | | | |
|---|---|---|---|
| | Minimum value | Maximum value | Mean |
| 30 Hz (R1) | 0,4 | 0,7 | 0,4727 |
| 70 Hz (R2) | 0,2 | 0,8 | 0,5824 |

| **Inspiration lead II** | | | |
|---|---|---|---|
| | Minimum value | Maximum value | Mean |
| 30 Hz (R1) | 0,2 | 1 | 0,4250 |
| 70 Hz (R2) | 0,2 | 1 | 0,68 |

| **Expiration lead I** | | | |
|---|---|---|---|
| | Minimum value | Maximum value | Mean |
| 30 Hz (R3) | 0,3 | 0,8 | 0,5333 |
| 70 Hz (R5) | 0,3 | 0,8 | 0,59 |

| **Expiration lead II** | | | |
|---|---|---|---|
| | Minimum value | Maximum value | Mean |
| 30 Hz (R3) | 0,3 | 0,8 | 0,5636 |
| 70 Hz (R5) | 0,4 | 1 | 0,7067 |

### Conclusions

Attempts were made to filter the interference with simple filters, but reliable results were not achieved. The interference consists of high frequencies but falls within the ECG frequency range (0-100 Hz), and therefore, we are losing essential ECG information. There are no very significant differences between 30 and 70 Hz, so the idea of changing the stimulation frequency is discarded, as it seems the generated noise cannot be avoided since it is embedded within the signal to record.

At first glance, it is apparent that interference has a higher amplitude with increased intensity. However, there is no difference in recovery times.

There is no difference in either amplitude or time when using different frequencies (30Hz and 70Hz); see Figures 4A to 4D. There are no amplitude differences between records 1, 3, and records 2, 4.

There is difference in amplitude between different leads (D.i and D.ii); there is more interference amplitude in DII. However, there is no change in recovery time (see results in the tables above).

There is no difference in either amplitude or time between different positions (inspiration and expiration).

The ECG recovery time, defined as the moment when the interference ceases to be maximal and the ECG signal begins to reappear until it is entirely free from interference, varies in each cardiac cycle but falls within the range of 200milliseconds to 1 second in extreme cases. With these values and assuming that we want to recover at least 2 respiratory cycles to calculate the heart rate, we propose a desirable minimum time between stimulations can be of 1 second plus 2 cardiac cycles, although some patients may achieve same effects with smaller times between stimulations, and some other biometric measurements do not require recovering any specific number of cycles, or may require other amount of cycles to perform some other measurements.

### Example 3: Analysis of the impact of electrical stimulation through transdiaphragmatic pressure.

The present study's ultimate objective is to quantitatively demonstrate the favourable effects of TERM in ICU patients. To achieve this, transdiaphragmatic pressure (Pdi) is measured as an indicator of inspiratory effort. Measurement of transdiaphragmatic pressure is the basis for assessment of diaphragmatic function as it represents the pressure across the diaphragm. During inspiration, the contraction of the diaphragm produces two effects: first, it pushes down on the viscera and displaces the abdomen outward: second, it reduces pleural pressure and thus inflates the lungs. The pressure generated by the diaphragm can be measured from simultaneous differences between oesophageal pressure (Pes) and gastric pressure (Pga) using a differential pressure transducer.

### Study design

The investigator screens 2 ICU patients for inclusion in the study as they meet the eligibility criteria collected in the protocol report. It is also important to note that all the patients were under controlled ventilation, which means that the ventilator was responsible for the entire respiratory process, and the subject's inspiratory effort was negligible.

The intervention consists of receiving thoracic and abdominal TERM at an intensity increased until visible muscular contraction or according to physician's criteria (to a maximum of 100 mA), with a frequency of 30 Hz and a pulse width of 350 us. The electrodes were placed to stimulate the inspiratory muscles:
- 2 electrodes in the *7th* intercostal space in the midaxillary line
- 2 electrodes in the paraxiphoid region

In each patient and for each recording, the stimulation intensity varies, tailored to the subject's anthropometric variables, with the aim of maximizing the effect while avoiding any discomfort. The following table displays the intensity values observed among the recordings:

| **Patient ID** | **Record 1 Intensity [mA]** | **Record 2 Intensity [mA]** | **Record 3 Intensity [mA]** | **Record 4 Intensity [mA]** | **RASS** | **fiO2** |
|---|---|---|---|---|---|---|
| Id1 | 20 | 40 | 60 | 60 | -5 | 0.35 |
| Id2 | 60 | 80 | 90 | 90 | -5 | 0.45 |

Each session will consist of obtaining 4 transdiaphragmatic pressure recordings from each patient with 4 minutes (240 seconds) of duration. Each Pdi recording will consist of 2 minutes without TERM and 2 minutes with TERM. During the 2 minutes with TERM the stimulating period is 10 seconds: 5 seconds of work and 5 seconds of rest which means a stimulating frequency of 0.1 Hz. Between recordings, there will be a washout period of, at least, 5 minutes to allow for Pdi amplitudes to return to basal values.

Relating with the analysis protocol, the aim is to measure the inspiratory effort defined as the inspiratory negative swing of the oesophageal pressure during tidal breathing. Transdiaphragmatic pressure will be obtained by simultaneously measuring oesophageal pressure and gastric pressure. Pdi will be calculated as the difference between Pga and Pes.

### Materials

NeuroTrac^{®} MyoPlus 2 (Verity Medical Ltd. Churchtown House Tagoat, Co. Wexford Ireland), was used for electrostimulation application.

-For the recordings of the variables of respiratory muscle effort during a 4-minute period the software FluxMed^{™}, MBMED Buenos Aires, Argentina, connected to the ICU ventilator was employed.

Esophageal and gastric balloons (Nutrivent, Sidam, Italy) were coupled to pressure transducers attached to the FluxMed^{™} monitoring system.

For the processing and analysis of the obtained data the software MATLAB (version R202.a, MathWorks Inc., Natick, MA, USA) was used.

### Methodology

The methodology starts with the time domain analysis of the signals with TERM (hereinafter referred to as the register number followed by 't') versus signals without TERM (hereinafter referred to as the register number without the 't' ).

After the observational analysis in the time domain a spectral analysis is followed. A spectral analysis is the process to decompose a signal into its frequency components, i.e., to examine which frequencies make up a signal. Spectral analysis helps in understanding the frequency content of a signal, identifying patterns, and extracting relevant information.

Based on the FFT results, high-pass filters (HPF), low-pass filters (LPF) and bandpass filters (BPF) have been used to select the frequencies of greatest interest.

Finally, the last method employed to obtain the desirable values is the window analysis, which enables the identification and quantification of changes or disturbances in the signal's critical path, as well as visualizing differences between classes or categories of signals. In this case, the window size is set at 15 seconds, aiming to encompass a complete respiratory cycle, including both inspiration and expiration. To account for variations in cycle length, as respiratory frequency can fluctuate slightly, a 3-second overlap is introduced between consecutive windows. This overlap ensures that no critical information is missed.

The primary goal of employing these methods is to analyse and compare the impact of TERM on the transdiaphragmatic pressure signal. Therefore, the subsequent phase of the study involves identifying disparities between signals with and without stimulation. Once these differences are established, the study aims to demonstrate that they indeed result in an increase in the mentioned pressure. This increase is quantified by calculating the transdiaphragmatic pressure delta (Δpdi), also referred to as transdiaphragmatic pressure swings. Δpdi is defined as the difference between the maximum and minimum pressure values during each inspiration, providing an index of respiratory effort.

### Results

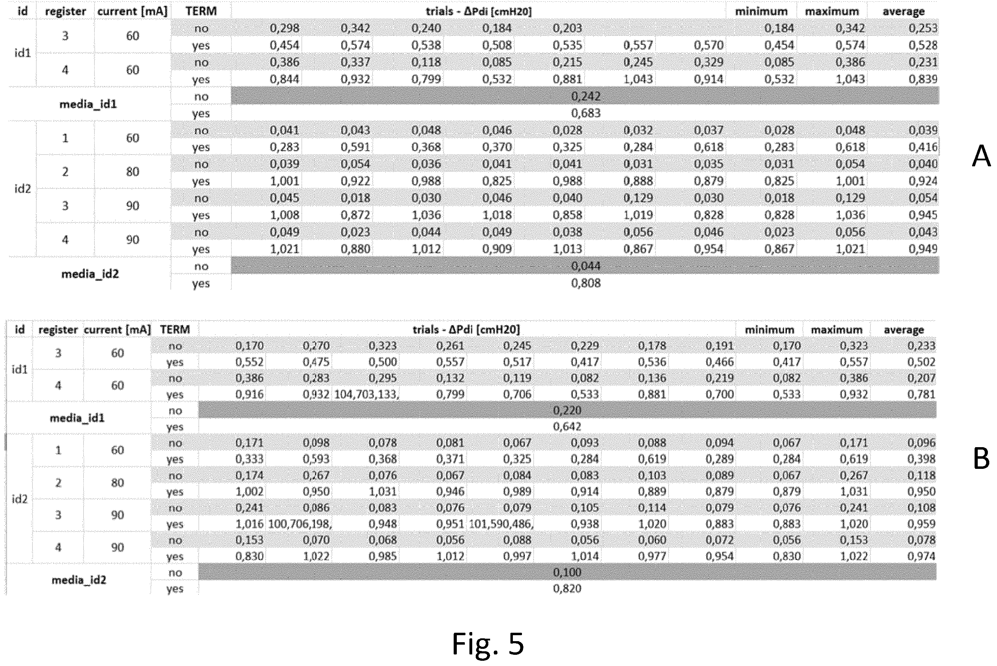

The first results obtained are summarized in Figures 5A and 5B in which the transdiaphragmatic pressure delta is calculated to compare the values between the recordings with and without TERM. It is worth noting that the word trials refers to each respiratory cycle during one recording.

Additionally, another informative analysis involves comparing the maximum Δpdi value with the initial Δpdi measurement taken without stimulation, also referred to as the basal measurement. This evolution can be observed in the following table, which records the maximum values achieved for each intensity, enabling us to assess the increment.

| **Id** | **Register** | **Basal measurement [cmH2O]** | **Highest measurement [cmH2O]** |
|---|---|---|---|
| Id1 | 3 | 0.184 | 0.574 |
| | 4 | 0.085 | 1.043 |
| Id2 | 1 | 0.028 | 0.618 |
| | 2 | 0.031 | 1.001 |
| | 3 | 0.018 | 1.036 |
| | 4 | 0.023 | 1.021 |

### Conclusions

The minimum stimulation intensity required for an optimal effect on the diaphragm is 60 mA, although it depends on the patient's body mass index. Although further studies with different patients would be desired, it seems that in any case the minimum intensity required for an optimal effect on the diaphragm would be less than 20mA.

When comparing signals without TERM to signals with TERM, we observe that the common factor in all records is frequency activity around 0.1 Hz (the frequency at which we are stimulating). If we isolate this activity, we find that it contributes to an increase in transdiaphragmatic pressure of approximately 1 cmH₂O.

### Example 4: Analysis of the use of multifield electrodes in a TERM system to determine the predetermined field and a current within a predetermined current range

The present study's ultimate objective is to determine a feasible method to determine the field and current range that generate a detectable respiratory muscle contraction without distortion or with the least distortion of one or more biometric measurements performed by one or more monitoring devices.

### Study design

### A) The multi-field electrodes

The multi-field electrode was divided into four multi-field surface electrodes applied to the subject's torso.

These electrodes, connected to an electrostimulator, were designed to direct electricity and assist patients in their inspirations and expirations. The multi-field electrode system allows for selective nerve stimulation, adapting to the individual needs of each subject.

Four electrodes were employed and each electrode has two groups of pads: anodes (current emitters) and cathodes (current receivers):
- Two electrodes for inspiration (one for each side of the torso) displayed in Figure 6A. These electrodes are designed to cover the anatomic area for stimulating the inspiratory muscles as described in literature.
- Two electrodes for expiration (one for each side) displayed in Figure 6B. These electrodes are designed to cover the anatomic area for stimulating the expiratory muscles as described in literature.

### B) Electrodes placement

Electrodes should be placed according to standard anatomical references. For inspiratory electrodes, the xiphoid process was used as a reference point, with the electrode extending towards the back to reach the intercostal area.

Expiratory electrodes should be placed with the cathode near the anterior superior iliac spine and diagonal to the xiphoid process. The anode of the expiratory electrodes was positioned on the posterolateral abdominal wall towards the 8th rib of the mid axillary line.

### C) Subjects

14 volunteer subjects were recruited for the study and all of them were provided detailed information about the study objectives and potential risks.

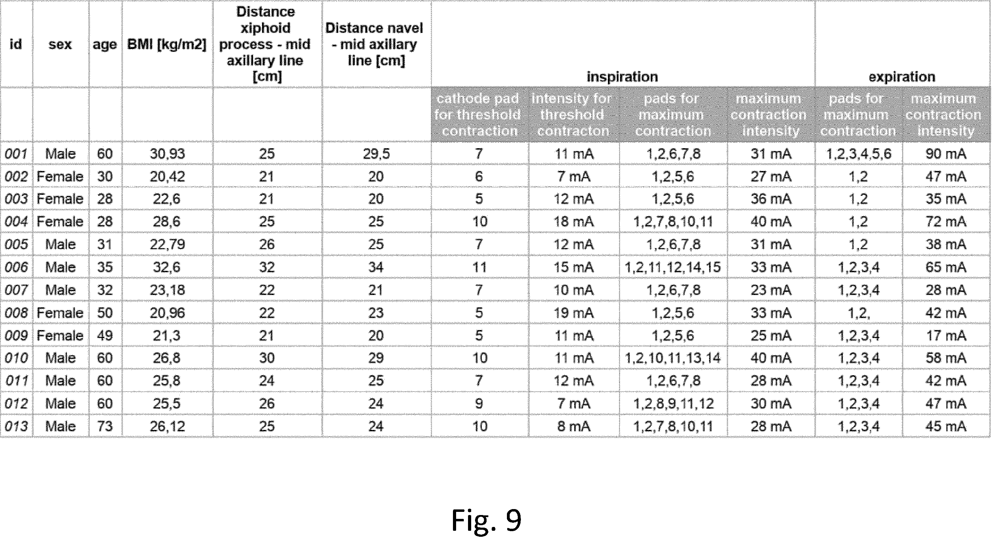

Figure 9 shows a table comprising information about the volunteers involved in the study. Their gender, age, body mass index (BMI), and relevant anthropometric measurements are included. The anthropometric measurements pertain to the anatomical area of interest and include the distance between the xiphoid process of the sternum and the mid-axillary line, as well as the distance between the navel and the mid-axillary line. The first measurement is associated with the inspiratory multi-field electrode (Figure 6A), and the second measurement is associated with the expiratory multi-field electrode (Figure 6B).

### D) Calibration

### - Calibration of the inspiration electrodes

To determine the field and current range to actuate the one or more inspiratory multifield skin electrodes, the following steps were taken:
a. stimulating a first cathode field of the electrode at a minimum current; wherein the minimum current is the minimum current as determined by the TERM system. In this case, the minimum current was set to 0mA.
b. increasing the current of step (a) until there is a detectable respiratory muscle contraction;
c. associating the current of step (b) to the cathode field of step (a);
d. repeating steps (a) to (c) for at least an additional cathode field of the one or more multifield skin electrodes (11a, 11b);
d1. selecting the cathode field with the minimum current required to generate a detectable respiratory muscle contraction as a minimised cathode field;
d2. increasing the area of the cathode field to be the same as the area of the anode field;
d3. repeating steps (a) to (c) for each cathode field of the one or more multifield skin electrodes (11a, 11b) comprising the minimised cathode field of step (d1).
e. determining the cathode field with the minimum current required to generate a detectable respiratory muscle contraction as the predetermined field, and determining the current required to generate a detectable respiratory muscle contraction in said predetermined field as the minimum of the predetermined current range.
f. stimulating the predetermined field determined in step (e) at the minimum of the predetermined current range as defined in step (e);
g. increasing the current of step (f) until the detectable respiratory muscle contraction does not increase further; and
h. determining the minimum intensity that provides the maximum detectable respiratory muscle contraction in said predetermined field as the maximum of the predetermined current range.

All of which are according to Fig. 7. Particularly, the following steps were taken:
1. Initially, the anode area was fixed by activating pads 1 and 2. Pads 3 and 4 were reserved for extreme cases of body mass index where contraction is insufficient with the established current amplitude limits.
2. The first cathode field to be checked was selected from those that fall within the described anatomical area: region between the 8th and 10th intercostal space at the mid-axillary line.
3. Calibration began with a current amplitude of 0 mA, gradually increasing until the first visible and palpable muscle contraction was detected, for example as determined by the expert operator, which was also tolerable for the subject. At this point, the current amplitude was recorded as the motor threshold (*I_{TH}*)*.*
4. Next, adjacent points are explored in ascending numerical order up to the same current intensity (*I_{TH}*) to identify the optimal placement of the cathode to access the motor point, where the minimum visible and palpable muscle contraction is generated. If a field is found that achieves contraction with a lower intensity, this is designated as the motor threshold field, and the new intensity is recorded as the new motor threshold (*I_{TH}*)*.*
5. Once the motor threshold pad is identified, combinations of pads around it are tested to form a 5x5 cm area, checking which one provides the greatest visible and palpable contraction with the lowest current intensity and greatest comfort (*I*_{C, INSP}).

For safety reasons, a maximum current amplitude limit of 90 mA was established. This amplitude is lower than the maximum current values of commercial electrostimulation equipment and was validated as a safety parameter by an expert in muscle electrostimulation.

Different combinations of stimulation were explored to identify the area that allows for selective contraction, prioritizing those pads that generate maximum visible and palpable muscle contraction. In the data collection notebook, the activated pads and the respective current intensity were recorded, both for the motor threshold (*I_{TH}*) and for the optimal contraction (*I*_{C, INSP} ).

### - Calibration of the expiration electrodes

To determine the field and current range to actuate the one or more expiratory multifield skin electrodes, the following steps were taken:
a. stimulating a cathode field of the electrode at a minimum current, wherein the minimum current is the minimum current as determined by the TERM system;
b. increasing the current of step (a) until a detectable respiratory muscle contraction does not increase further; and
c. determining the minimum intensity that provides a detectable respiratory muscle contraction does not increase further as the maximum of the predetermined current range.

All of which are according to Fig. 8.

In this case, depending on the subject's BMI, the anode and cathode fields were selected seeking the maximum contraction of the expiratory muscles (posterolateral abdominal wall), so that they contract creating the so-called "wasp waist".

### Materials and methods

To execute the study plan, the following materials were used:
- Stimulator: STIM 2.1. Stimulator design by Tecnalia or Myoplus Neurotrac stimulator.
- Multifield electrodes: built in house by TESAI CARE S.L.
- Control system: a demultiplexing plate between electrodes and stimulator to decide which pads activate.
- Hydrogel: Axelgaard hydrogel AG2530/AG735.

In all cases the relationship between threshold contraction (minimum current required to generate a detectable respiratory muscle contraction) and maximum contraction intensities to assess whether there is a relationship between these two values.

### Results

The results of the predetermined electrodes and predetermined maximum intensity range can be found in Fig. 9.

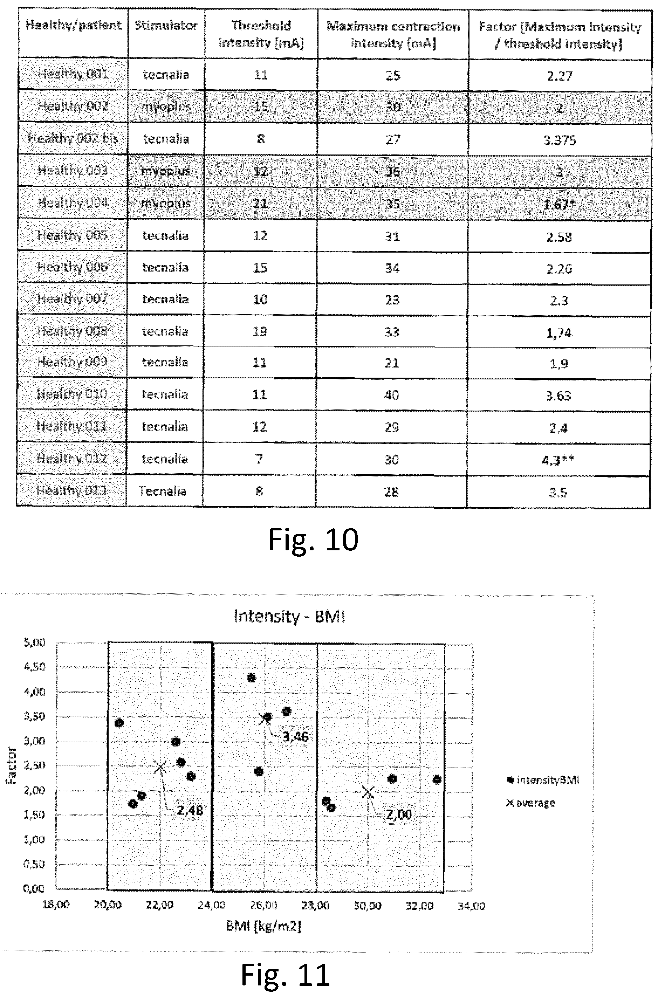

The results of the study between the relationship between threshold intensity and maximum contraction intensities can be found in Fig. 10.

### Conclusions

The proposed methodology is able to determine a field (pad or group of pads) within a multifield electrode and a current range to actuate to actuate the one or more multifield skin electrodes for a certain patient that that generate a detectable respiratory muscle contraction without distortion or with the least distortion of one or more biometric measurements performed by one or more monitoring devices.

Moreover, Fig. 11 shows a graph that represents the relationship factor between the threshold intensity and maximum contraction intensities of all patients for each body mass index. If the BMI were not to be considered, the mean value would be 2.58, with a maximum value of 4.3 and a minimum of 1.67. Therefore, using a factor of 2.58 for everyone would be very risky as it could be uncomfortable in some cases and ineffective in others. To try to personalize this value by ranges, a scatter plot was created and the mean was calculated for each range: for example, BMI under 24, between 24 and 28, and over 28. Thus, the means are more personalized.

It is noted that the methodology herein described is according to one or more embodiments of the invention.

### Example 5: Clinical concept validation on healthy volunteer: effect of TERM on diaphragm function

### 1. Study Justification and Objective

The objective of this study is to demonstrate that transcutaneous electrostimulation of the respiratory muscles (TERM) activates the diaphragm muscle. This is evaluated using ultrasound imaging and the analysis of pressure signals, both gastric and esophageal.

### 2. Study Design

In this study, transcutaneous electrical stimulation was applied using specific parameters: rectangular, biphasic, and compensated pulses at a frequency of 30 Hz, with a pulse width of 350 microseconds. The current intensity was adjusted to achieve a visible and palpable muscle contraction. The duration of stimulation varied depending on the device used.

When using the commercial Neurotrac^{®} Myoplus stimulator, the ON and OFF times were set manually by the user, allowing for either unilateral or bilateral stimulation. Conversely, when using the Heecap^{®} device, two stimulation modes were available:
- Bilateral stimulation: This required the Heepsync algorithm, which relies on flow and pressure signal readings. However, as the subject was a healthy volunteer without tubing, this option was not feasible.
- Unilateral stimulation: This involved manually activating and deactivating the channels using the ON/OFF button of the device.

The electrodes employed varied based on the test. Both conventional electrodes for electrical stimulation and multifield electrodes designed by Heecap were used, the latter facilitating the mapping process.

The study was conducted in two phases: the first focused on obtaining ultrasound images, while the second involved recording pressure signals.

### 2.1. Ultrasound imaging technique

Ultrasound imaging (US) is widely used in studies evaluating diaphragm muscle contraction as it allows for the visualization of muscle thickness. In this study, M-mode US was employed, which provides an image over time, resembling a video that captures the muscle's contraction dynamics. M-mode US imaging reflects motion of connective tissue within muscles and it may be used to measure non-invasively the onset of deep muscle activity.

This technique is advantageous due to its non-invasive nature and ease of implementation. However, it has limitations, including the lack of standardization, which introduces both inter-subject and intra-subject variability in measurements.

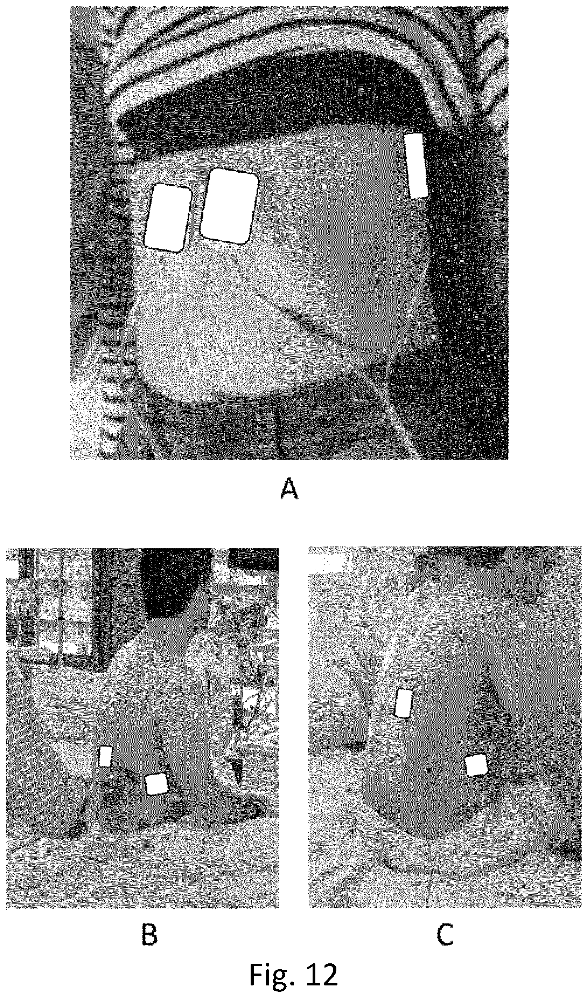

Using this technique, several tests were conducted by varying the position of the ultrasound probe and the location of the stimulation electrodes. The specific designs of these tests are described as follows:
- **Test 1:** Measuring the ultrasound signal on the right front-lateral side using electrode position 1, as shown in Figure 12A. This electrode placement corresponds to the conventional positioning commonly referenced in the literature to stimulate the diaphragm and intercostal muscles (Hsin YF, Chen SH, Yu TJ, Huang CC, Chen YH. Effects of transcutaneous electrical diaphragmatic stimulation on respiratory function in patients with prolonged mechanical ventilation. Ann Thorac Med 2022;17:14-20.) The electrodes are placed to stimulate the inspiratory muscles:
   o - 2 electrodes bilaterally in the 7th intercostal space in the midaxillary line
   o - 2 electrodes bilaterally in the paraxiphoid region
- **Test 2:** Measuring the ultrasound signal on the back of the body with electrode placement as indicated in Figure 12A, position 1.
- **Test 3:** Measuring the ultrasound signal on the front of the body with electrode placement as indicated in Figure 12A, position 1. This test objective was to measure the diaphragmatic excursion.
- **Test 4:** Measuring the ultrasound signal on the back of the body while adjusting the electrode placement as shown in Figure 12B, position 2. This second position was used in an attempt to focus the electrical stimulation on the back portion of the diaphragm to evaluate its contraction. The electrode located near the xiphoid process was moved to the back seeking the 7th posterior intercostal space and next to the spinal column. and the amplitude of the current was changed to 25 mA. Also, the subject was sitting down, not laying.
- **Test 5:** Measuring the ultrasound signal on the back of the body while further adjusting the electrode placement to position 3, as shown in Figure 12C, located more towards the back and upper region, specifically in the 5th posterior intercostal space.. This adjustment was again aimed at specifically targeting the back of the diaphragm muscle.

The following table summarizes the differences between the tests conducted to obtain the ultrasound images. In all cases, the Heecap^{®} device was used as the stimulator, meaning the stimulation was unilateral for all tests. Additionally, conventional electrodes were employed throughout.

**Table 1. Overview of the different tests performed to obtain ultrasound images**

| **Test** | **US probe position** | **Stimulating electrodes configuration** | **Stimulator** | **Electrodes employed** |
|---|---|---|---|---|
| Test 1 | Front Lateral | Position 1 | Heecap^{®} | Conventional |
| Test 2 | Back | Position 1 | Heecap^{®} | Conventional |

| Test 3 (diaphragmatic excursion) | Front | Position 1 | Heecap^{®} | Conventional |
|---|---|---|---|---|
| Test 4 | Back | Position 2 | Heecap^{®} | Conventional |
| Test 5 | Back | Position 3 | Heecap^{®} | Conventional |

### Diaphragm thickening fraction

The diaphragm thickening fraction (TFdi) is a surrogate measure of inspiratory effort ([7] Goligher EC, Dres M, Fan E, Rubenfeld GD, Scales DC, Herridge MS, Vorona S, Sklar MC, Rittayamai N, Lanys A, Murray A, Brace D, Urrea C, Reid WD, Tomlinson G, Slutsky AS, Kavanagh BP, Brochard LJ, Ferguson ND. Mechanical Ventilation-induced Diaphragm Atrophy Strongly Impacts Clinical Outcomes. Am J Respir Crit Care Med. 2018 Jan 15;197(2):204-213. doi: 10.1164/rccm.201703-0536OC. PMID: 28930478.) and for its measurement images are obtained during 2 situations:
1. Spontaneous breathing: breathing at a normal rate. The muscle thickness is calculated with the following formula: $\begin{matrix}TFdi \left(physiological breathing\right) \\ = \frac{thickness at the end of the inspiration - thickness at the end of the expiration}{thickness at the end of the expiration} ⋅ 100 %\end{matrix}$ It is worth mentioning that the end of the expiration corresponds to the basal measurement since it could be defined as a resting state. Therefore, in this report basal measurement and measurement at the end of expiration will be used indistinctly.
2. Diaphragmatic stimulation: during TERM the calculation is done in expiration apnea and using the following equation: $\begin{matrix}TFdi \left(TERM\right) = \frac{thickness during stimulation - thickness at the end of the expiration}{thickness at the end of the expiration} \\ ⋅ 100 %\end{matrix}$ Again 'thickness at the end of the expiration' corresponds to the basal value of thickness at a resting state, whereas the measure of 'thickness during stimulation' is the thickness of the diaphragm achieved when the contraction is caused by the electrical stimulus.

### Diaphragmatic excursion

The diaphragmatic excursion is the measurement of the amplitude of the diaphragm displacement between inspiration and expiration phases. It could be measured using the anterior subcostal view with the convex probe positioned below the costal margin between the midclavicular line and anterior axillary line (Santana PV, Cardenas LZ, Albuquerque ALP, Carvalho CRR, Caruso P. Diaphragmatic ultrasound: a review of its methodological aspects and clinical uses. J Bras Pneumol. 2020 Nov 20;46(6):e20200064. doi: 10.36416/1806-3756/e20200064. PMID: 33237154; PMCID: PMC7909996.).

### 2.2. Pressure signals recording: esophageal and gastric

In the second phase, pressure and gastric signals were analyzed to confirm diaphragm activation.

Measurement of transdiaphragmatic pressure (Pdi) is the basis for assessment of diaphragmatic function as it represents the pressure across the diaphragm. It is calculated with the following formula: $Pdi = Pga - Pes$

During inspiration, the contraction of the diaphragm produces two effects: first, it pushes down on the viscera and displaces the abdomen outward: second, it reduces pleural pressure and thus inflates the lungs.

The pressure generated by the diaphragm can be measured from simultaneous differences between esophageal pressure (Pes) and gastric pressure (Pga) using a differential pressure transducer (Brochard, L. (1991). Transdiaphragmatic Pressure. In: Benito, S., Net, A. (eds) Pulmonary Function in Mechanically Ventilated Patients. Update in Intensive Care and Emergency Medicine, vol 13. Springer, Berlin, Heidelberg. https://doi.org/10.1007/978-3-642-84209-2_5). This methodology is well-established, with consistent agreement regarding the conclusions drawn from the pressure signals. However, its major limitation is that it requires the subject to swallow a catheter, making it challenging for healthy subjects, though it may be more feasible in patients who already have a catheter in place.

In this study the double balloon catheters were introduced through the nose of the subject. The objective was to stimulate and check gastric and esophageal pressure with the ventilator since the Fluxmed device was not working so we connected to the ventilator a transducer to obtain the measurements of those pressures on the ventilator.

To obtain these signals the following tests have been done:
- Test 1: Stimulating with Heecap^{®}stimulator device and multifield electrodes.
   o **For this** test the current intensity was increased until 32 mA, and the activated pads for the inspiration multifield electrodes were 1,2 from the anode and 7,8,10,11 for the cathode (see Figure 6A).
- Test 2: Stimulating with the commercial Neurotrac^{®} Myoplus electrostimulator and conventional electrodes.
   ∘ For this second test the stimulator was changed to the commercial one so we could stimulate bilaterally because maybe stimulating on both sides the difference in Pdi was more visible. The ON parameter of the stimulator was set to 1 second and 1 seconds OFF time. Since the commercial stimulator does not work with multifield electrodes because of an impedance problem, the electrodes were also changed to the commercial ones. Also, an abdominal band was put to avoid the contraction of the abdominal wall and to have a more pronounced signal in Pga.

The process was: couple deep breaths and in the third breath the subject was asked to expire slowly trying to maintain an apnea during5 stimulating cycles (1 second ON + 1 second OFF).

**Table 2. Overview of the different tests performed to obtain pressure and gastric signals**

| **Test** | **Stimulator** | **Electrodes** |
|---|---|---|
| Test 1 | Heecap^{®} | Multifield |
| Test 2 | Neurotrac^{®} Myoplus | Conventional |

### 3. Materials

- NeuroTrac^{®} MyoPlus (Verity Medical Ltd. Churchtown House Tagoat, Co. Wexford Ireland).
- Heecap^{®} V0 prototype device
- For the recordings of the variables of respiratory muscle effort the softwareFluxMed^{™}, MBMED Buenos Aires, Argentina.
- Philips CX-50 ultrasound scanner (Koninklijke Philips N.V., Amsterdam, Netherlands)
- Esophageal and gastric balloons (Nutrivent, Sidam, Italy).
- Abdominal band
- Self-adhesive electrodes DURA-STICK^{®} (Chattanooga, Barcelona, Spain)
- Heecap^{®} multifield electrodes (Figure 6A)

### 4. Data analysis

### 4.1. Ultrasound images

### • Test 1: Measuring the ultrasound signal on the right front-lateral side using electrode position 1.

Several iterations have been made, also changing the current intensity of stimulation.

The first iteration is made with 26 mA of current intensity. Following the equations 1 and 2, the results for this iteration are: $TFdi \left(physiological breathing\right) = \frac{0.56 cm - 0.18 cm}{0.18 cm} ⋅ 100 % = 2.11 ⋅ 100 % = 211 %$ $TFdi \left(TERM\right) = \frac{0.28 cm - 0.18 cm}{0.18 cm} ⋅ 100 % = 0.56 ⋅ 100 % = 56 %$

A second iteration of the ultrasound image stimulating with 28 mA have the following results: $TFdi \left(TERM\right) = \frac{0.249 cm - 0.257 cm}{0.257 cm} ⋅ 100 % = 0 ⋅ 100 % = 0 %$

In this second iteration, there is more abdominal contraction than diaphragmatic, so it is worse.

For the third iteration the intensity was decreased to 24 mA and the results obtained were: $TFdi \left(TERM\right) = \frac{0.296 cm - 0.28 cm}{0.28 cm} ⋅ 100 % = 0.057 ⋅ 100 % = 5.7 %$

In the last iteration with also 24 mA the diaphragm thickening is the following. $TFdi \left(TERM\right) = \frac{0.288 cm - 0.202 cm}{0.202 cm} ⋅ 100 % = 0.43 ⋅ 100 % = 43 %$

### • Test 2: Measuring the ultrasound signal on the back of the body with electrode placement as position 1.

This second test is performed because the contraction of the diaphragm in the back is also wanted to be probed since E.Goligher feels the contraction more on the front and not on the back, and the diaphragm is longer on the back so most physiological contraction comes from there.

However, with these tests the contraction of the diaphragm on the back was not possible to be seen. The reason for this is because when breathing in, the lung shadow masked the diaphragm in the eco on the back. With stimulation we do not see thickening in the diaphragm. It is more interior costal contraction.

### • Test 3: Measuring the ultrasound signal on the front of the body with electrode placement as position 1.

The excursion of the diaphragm was tried to be measured in the front. However, the abdominal muscle contraction shifts the probe up and difficulties the diaphragmatic excursion visualization.

### • Test 4: Measuring the ultrasound signal on the back of the body while adjusting the electrode placement as position 2.

$TFdi \left(TERM\right) = \frac{0.288 cm - 0.202 cm}{0.202 cm} ⋅ 100 % = 0.43 ⋅ 100 % = 43 %$

In view of the results obtained with this configuration of stimulating electrodes, E. Goligher does not think there is some thickening. However the position of the electrode is not certain, since this configuration is not mentioned in bibliography, therefore the motor point could be far from the point of stimulation.

### • Test 5: Measuring the ultrasound signal on the back of the body while further adjusting the electrode placement to position 3.

This last test is just moving the stimulating electrodes a bit more up on the back trying to better access the motor point, but it seems that the stimulation contracts more the abdominal muscle than diaphragm. The result for this last test is: $TFdi \left(TERM\right) = \frac{0.272 cm - 0.218 cm}{0.218 cm} ⋅ 100 % = 0.25 ⋅ 100 % = 25 %$

### 4.2. Pressure signals data

### • Test 1: Pressure measure with double balloon esophagic and gastric, Heecap^{®} and multifield electrodes.

The observation of the first iteration of this test with 32 mA was that if there was something it was very slender, no video or data was registered during this test.

For a second iteration of this test the current intensity was decreased to 29 mA. After checking the pressure, we conclude that Pdi was actually going down during stimulation, maybe because of the contraction of the chest wall (because Pga goes down and Pes goes up) as when you cough. This means that there was no diaphragm contraction or at least not visible, maybe because we were stimulating just on one side so the changes on Pdi were not visible.

### Test 2: Pressure measure with double balloon esophagic and gastric, commercial electrostimulator (Neurotrac Myoplus) and conventional electrodes. The current was increased until 25 mA.

In view of the curves observed, Pga goes up more or less 5 mmHg, which corresponds to 7 cmH20, and Pes decreases to approximately -2 cmH20, therefore there is an increase of Pdi of 9 cmH20, since Pdi is Pga - Pes. Pga goes up so there is a diaphragm contraction and Pes goes down or stays the same.

### 5. Results

The results of the TF measured with the ultrasound technique can be seen in Table 3.

**Table 3. Results of the diaphragm thickening fraction obtained from US images with electrostimulation**

| Test | Iteration | Diaphragm thickening fraction |
|---|---|---|
| Test 1 | 1 | 56% |
| | 2 | 0% |
| | 3 | 5.7% |
| | 4 | 43% |
| Test 2 | 1 | No results |
| Test 3 | 1 | No results |
| Test 4 | 1 | 0% |
| Test 5 | 1 | 25% |

And the results for the measured pressures are shown in the following table.

**Table 4. Results of the gastric, esophageal and transdiaphragmatic pressure measurements while applying electrostimulation of respiratory muscles.**

| **Test** | **Pga** | **Pes** | **Pdi (Pga-Pes)** |
|---|---|---|---|
| Test 1 | No results | No results | No results |
| Test 2 | 7cm H₂O | -2cm H₂O | 9cm H₂O |

### 6. Conclusions

Demonstrating diaphragm contraction using ultrasound imaging is a challenging task, especially for the posterior portion of the diaphragm, due to the lack of standardization in measurement techniques.

Several publications [8] mentioned that TF < 20% for mechanically ventilated patients could be associated with longer stay in intensive care units, prolonged mechanical ventilation and increased mortality.

According to E. Goligher, the observed results of the tests with less than 20% of thickening could be attributed to image resolution issues, making the measurement less objective. Furthermore, the unilateral stimulation applied during these tests may not have been sufficient to generate a noticeable diaphragm contraction. On the other hand, thickening fractions higher than 20% - test 1 iteration 1 and 4, and test 5 - could be described as successful results. Given the inherent difficulties of this technique, additional tests are required to identify the optimal method for measuring diaphragm thickening during transcutaneous electrical respiratory muscle stimulation.

On the other hand, the pressure signal results provide stronger evidence of diaphragm contraction. E. Goligher noted that an increase in transdiaphragmatic pressure (Pdi) - indicated by a rise in gastric pressure (Pga) - is consistent with diaphragm activation.

In conclusion, this study suggests that Pdi signals are a more reliable method than ultrasound imaging for evaluating diaphragm contraction. Most importantly, it demonstrates that transcutaneous electrostimulation can successfully activate the diaphragm muscle.

### Example 6: Clinical concept validation on critical patients within ICU environment

### Materials and methods

As a preliminary phase of a pilot study, two ICU critical patients underwent synchronized respiratory muscular electrical stimulation through a system according to one or more aspects of the invention. The system comprised:
- a transcutaneous electrostimulation of the respiratory muscles (TERM) system comprising one or more skin electrodes configured to be placed over the respiratory muscles of the patient;
- at least one sensor for sensing the breathing state of the patient; and
- a processor configured to receive the breathing state of the patient from the at least one sensor, and actuate the TERM system when the breathing state is in the inspiration phase;

The one or more skin electrodes comprising an anode and a cathode.

The processor was configured to actuate the one or more multifield skin electrodes with the following current intensities

| | **day 1** | | **day 2** | |
|---|---|---|---|---|
| **Patient** | **Inspiratory intensity (mA)** | **Expiratory intensity (mA)** | **Inspiratory intensity (mA)** | **Expiratory intensity (mA)** |
| p0001 | 40 | 50 | - | - |
| p0002 | 30 | - | 20 | 40 |

In order to evaluate the system, the following parameters were established:
- True Positive (TP): A respiratory event (inspiration or expiration) that was correctly stimulated - stimulation was needed and appropriately delivered
- False Positive (FP): A respiratory event (inspiration or expiration) that was incorrectly stimulated - stimulation was applied when it was not needed.
- True Negative (TN): A respiratory event (inspiration or expiration) that was correctly not stimulated - no stimulation was needed, and none was delivered.
- False Negative (FN): A respiratory event (inspiration or expiration) that was incorrectly not stimulated - stimulation was needed but not delivered.

### Results

• Inspiration

| | | Day 1 | | | Day 2 | | |
|---|---|---|---|---|---|---|---|
| Hospital | Patient | # Stimulations | FP | % TP Stimulations | # Stimulations | FP | % TP Stimulations |
| tauli | p0001 | 211 | 1 | 99.53% | | | |
| tauli | p0002 | 338 | 18 | 94.67% | 192 | 3 | 98.44% |

• Expiration

| | | Day 1 | | | Day 2 | | |
|---|---|---|---|---|---|---|---|
| Hospital | Patient | # Stimulations | FP | % TP Stimulations | # Stimulations | FP | % TP Stimulations |
| tauli | p0001 | 190 | 0 | 100.00% | - | - | - |
| tauli | p0002 | - | - | - | 182 | 0 | 100.00% |

The percentage of true positives (TP) of patient p0001 was 99.77% while for patient p0002 was 97.70%.

Additionally a medical expert evaluated the breathings of the signals and the following results were obtained

A relative-based functioning algorithm (validated by medical doctors) was used to validate the same breathings, with the following results:

| **OUTPUT** | |
|---|---|
| **TN** | 29 |
| **TP** | 64 |
| **FN** | 1 |
| **FP** | 6 |
| **Sensibility** | 98.46% |
| **Specificity** | 82.86% |

## Claims

1. A system (100) for synchronized respiratory muscular electrical stimulation of a patient, the system comprising:
- a transcutaneous electrostimulation of the respiratory muscles (TERM) system (10) comprising one or more skin electrodes (11a, 11b) configured to be placed over the respiratory muscles of the patient;
- at least one sensor (20) for sensing the breathing state of the patient; and
- a processor (30) configured to receive the breathing state of the patient from the at least one sensor (20), and actuate the TERM system (10) when the breathing state is in the inspiration phase;
wherein each of the one or more skin electrodes (11a, 11b) are multifield electrodes comprising an anode and a cathode;
wherein the processor (30) is further configured to actuate the one or more multifield skin electrodes (11a, 11b) at a current intensity of at least 20 mA.

2. The system according to claim 1, wherein the processor (30) is further configured to actuate the one or more multifield skin electrodes (11a, 11b) at a current intensity comprised between 20 mA and 100 mA.

3. The system according to one of the previous claims, wherein the processor is further configured to actuate the one or more multifield skin electrodes at a current intensity comprised between 20 mA and 60 mA.

4. The system according to any one of the previous claims, wherein the processor (30) is further configured to actuate the TERM system (10) when the breathing state is in the expiration phase.

5. The system according to claim 4, wherein when the processor is configured to actuate the TERM system when the breathing state is in the expiration phase, the processor is further configured to actuate the one or more multifield skin electrodes at a current intensity comprised between 40 mA and 100 mA

6. The system according to any one of the previous claims, wherein the system (100) further comprises at least one electromyography (EMG) sensor (40), the at least one EMG sensor (40) configured to measure the activity of one or more of the respiratory muscles of the patient, preferably wherein the at least one EMG sensor (40) is a superficial EMG sensor; and
wherein the processor (30) is further configured to actuate the TERM system (10) considering the activity of one or more of the respiratory muscles of the patient as measured by the EMG sensor.

7. The system according to claim 6, wherein the processor (30) is further configured to determine the fatigue of the respiratory muscles of the patient from the activity registered by the at least one EMG sensor; and
wherein the processor (30) is further configured to actuate the TERM system (10) considering the fatigue status of the respiratory muscles of the patient.

8. The system according to any one of the previous claims, wherein the TERM system (10) is configured to apply at least two electrical stimuli with an intensity that distorts one or more biometric measurements performed by one or more monitoring devices and with a period between consecutive electrical stimuli of at least 200 milliseconds.

9. The system according to claim 8, wherein the one or more biometric measurements require a minimum measurement period to obtain a parameter of interest and wherein the period between consecutive electrical stimuli is extended by an additional period corresponding to the measurement period the one or more biometric measurements require.

10. The system according to claim 8 or 9, wherein the intensity that distorts the one or more biometric measurements performed by one or more monitoring devices is at least of 20mA.

11. The system according to any one of the claims 8 to 10, wherein the biometric measurement is an electrocardiogram and the monitoring device an electrocardiogram monitoring device.

12. The system according to any one of the previous claims, wherein the actuation of the TERM system is further adapted based on mechanical ventilated-related variables, preferably wherein the mechanical ventilated-related variables comprise any of: airway pressure (Paw), flow, and volume pressure.

13. The system according to any one of the previous claims, wherein the TERM system is configured to actuate the one or more multifield skin electrodes (11a, 11b) using an anode field and cathode field with an area of between 10 and 50 cm².

14. A computer implemented method for synchronized electrical stimulation with a system (100) for synchronized respiratory muscular electrical stimulation of a patient according to any one of claims 1 to 13, the method comprising:
a) receiving information regarding the breathing state of the patient from a sensor (20) connected to a mechanical ventilator,
b) determining from the information if the breathing state corresponds to a selected breathing phase of the patient;
c) when the breathing state corresponds to the selected breathing phase of the patient, indicating to the processor (30) that the breathing state corresponds to the selected breathing phase of the patient;
wherein the selected breathing phase is selected from any of: inspiration phase and/or expiration phase.

15. A computer program product comprising instructions configured to perform the method according to claim 14.
